# EUROPÄISCHE PATENTSCHRIFT

(11) **EP 1 523 984 B1**
(45) Veröffentlichungstag und Bekanntmachung des Hinweises auf die Patenterteilung: **06.12.2006**
(21) Anmeldenummer: 03090343.9
(22) Anmeldetag: 13.10.2003
(51) Int. Cl.: A61K 33/24, A61P 35/00

(54) **Pharmazeutische Zusammensetzung umfassend Oxoplatin und dessen Salze**
Pharmaceutical composition comprising oxoplatin and salts thereof
Compositions pharmaceutiques comprenant l'oxoplatine et ses sels

(43) Veröffentlichungstag der Anmeldung: 20.04.2005
(73) Patentinhaber: Salama, Zoser B. nat.rer.Dr., 13465 Berlin (DE)
(72) Erfinder: Salama, Zoser B. nat.rer.Dr., 13465 Berlin (DE)
(74) Vertreter: Hertin, Paul W.

(56) Entgegenhaltungen:
- EP-A- 0 339 772
- WO-A-03/066526
- RU-C- 2 086 261
- US-A- 4 119 653
- PRESNOV, M. A. ET AL: "The antitumor activity of oxoplatinum" NEOPLASMA (1985), 32(1), 73-83 , XP008027150
- PRESNOV, M. A. ET AL: "Antitumor properties of cis-dichlorodiamminedihydroxyplatinum(IV)" IZVESTIYA AKADEMII NAUK SSSR, SERIYA BIOLOGICHESKAYA (1986), (3), 417-28 , XP008027144
- ORR, R. M. ET AL: "Evaluation of novel platinum (II), and platinum (IV) ammine/amine complexes in L1210 murine leukemia cell lines sensitive and resistant to cisplatin and tetraplatin" CELLULAR PHARMACOLOGY (1993), 1(1), 17-23 , XP008027140
- BRANDON R J ET AL: "Synthesis, characterization, and properties of a group of platinum (IV) complexes." JOURNAL OF MEDICINAL CHEMISTRY. UNITED STATES JUL 1984, Bd. 27, Nr. 7, Juli 1984 (1984-07), Seiten 861-865, XP001184796 ISSN: 0022-2623
- PRESNOV, M. A. ET AL: "Cycloplatam and oxoplatin - the new antitumor platinum compounds of the second generation" ARCHIV FUER GESCHWULSTFORSCHUNG (1988), 58(1), 43-9 , XP008027148
- KELLAND, L. R. ET AL: "Structure-activity relationships in a series of novel platinum(II) and platinum(IV) ammine-amine complexes evaluated against a panel of human ovarian carcinoma cell lines" JOURNAL OF CELLULAR PHARMACOLOGY (1992), 2(6), 331-42 , XP008027138
- KEPRTOVÁ J ET AL: "The effect of second generation platinum cytostatics on mammalian cell proliferation." NEOPLASMA. CZECHOSLOVAKIA 1990, Bd. 37, Nr. 2, 1990, Seiten 121-129, XP008027200 ISSN: 0028-2685
- BLATTER E E ET AL: "Interaction of the antitumor agents cis,cis,trans-PtIV(NH3)2Cl2(OH)2 and cis,cis,trans-PtIV[(CH3)2CHNH2]2Cl2(OH)2 and their reduction products with PM2 DNA." BIOCHEMISTRY. UNITED STATES 9 OCT 1984, Bd. 23, Nr. 21, 9. Oktober 1984 (1984-10-09), Seiten 4817-4820, XP001184794 ISSN: 0006-2960
- KONOVALOVA, A. L. ET AL: "Antineoplastic effect of complex platinum(IV) compounds" DOKLADY AKADEMII NAUK SSSR (1977), 234(1), 223-6 [BIOCHEM.] , XP008027146
- YEN, TRAN CONG ET AL: "Study on potential of prolongation of survival in mice with cancers (before and after amputation) treated with cis-dichlorodiamine trans-dihydroxo platinum(IV)" TAP CHI DUOC HOC (2001), (2), 19-21 , XP001184196
- TRAN, CONG YEN ET AL: "Action of platinum(IV) complexes on sarcoma TG-180 cells in vivo" TAP CHI DUOC HOC (1998), (6), 18-20 , XP001184197
- NGUYEN, THI QUY ET AL: "The antitumor effectiveness of a platinum(IV) compound in Swiss mice" TAP CHI DUOC HOC (1998), (3), 21-23 , XP001184198
- AREF'EVA A K ET AL: "[Antitumor effectiveness and nephrotoxicity of oxoplatinum]" VOPROSY ONKOLOGII. USSR 1990, Bd. 36, Nr. 3, 1990, Seiten 331-334, XP008027204 ISSN: 0507-3758
- KELLAND L R ET AL: "A novel trans-platinum coordination complex possessing in vitro and in vivo antitumor activity." CANCER RESEARCH. UNITED STATES 1 NOV 1994, Bd. 54, Nr. 21, 1. November 1994 (1994-11-01), Seiten 5618-5622, XP001161097 ISSN: 0008-5472
- TOBE M L ET AL: "Structure, activity, reactivity and solubility relationships of platinum diamine complexes" J.CLIN.HEMATOL.ONCOL. 1977, Bd. 7, Nr. 1, 1977, Seiten 114-137, XP008027197
- VOLLANO J F ET AL: "DNA BREAKAGE BY A PERHYDRATE COMPLEX OF CIS,CIS,TRANS-PTIVCL2(NH3)2(OH)2" JOURNAL OF THE AMERICAN CHEMICAL SOCIETY, XX, XX, Bd. 106, Nr. 9, 1984, Seiten 2732-2733, XP001187504 ISSN: 0002-7863
- NOVAKOVA, OLGA ET AL: "DNA interactions of antitumor platinum(IV) complexes" EUROPEAN JOURNAL OF BIOCHEMISTRY (1995), 228(3), 616-24 , XP008027141
- BRABEC V ET AL: "TETRAVALENT PLATINUM COMPLEXES CAN EXERT THEIR ANTITUMOR EFFECT VIA DIRECT REACTION WITH DNA" STUDIA BIOPHYSICA, Bd. 114, Nr. 1-3, 1986, Seiten 199-207, XP008027208 7TH CMEA (COUNCIL ON MUTUAL ECONOMIC AID) SYMPOSIUM ON BIOPHYSICS OF NUCLEIC ACIDS AND PROTEINS, BRN ISSN: 0081-6337
- GUTSCHE, W. ET AL: "Structure-activity relationships of active antineoplastic platinum(II) an (IV) coordination compounds" ARCHIV FUER GESCHWULSTFORSCHUNG (1989), 59(4), 233-8 , XP008027147
- GIANDOMENICO C M ET AL: "CARBOXYLATION OF KINETICALLY INERT PLATINUM(IV) HYDROXY COMPLEXES. AN ENTREE INTO ORALLY ACTIVE PLATINUM(IV) ANTITUMOR AGENTS" METAL CONSTRUCTION, CAMBRIDGE, GB, Bd. 34, 1995, Seiten 1015-1021, XP001005596

## Beschreibung

Die Erfindung betrifft ein pharmazeutisches, insbesondere ein chemotherapeutisches Mittel, umfassend cis-Diammoniumdichlorotransdihydroxoplatin (IV) in Form von Kapseln, Tabletten, Cremes, Salben und Infusionslösungen, sowie die Herstellung dieser pharmazeutischen Mittel; die Erfindung betrifft auch die Verwendung der pharmazeutischen Mittel zur Behandlung von Tumorerkrankungen.

Bei Krebs handelt es sich um das unkontrollierte Wachstum von neuem Gewebe, das durch Entartung körpereigener Zellen verursacht wird. Die Krebszellen besitzen die Fähigkeit, in anderes Gewebe einzudringen und dieses zu zerstören. Zu den Hauptgruppen der Krebserkrankung bzw. der Tumorerkrankungen gehören Sarkome, Karzinome, Leukämien und Lymphome. In den Industriegesellschaften gehören Krebs- und Tumorerkrankungen zu den häufigsten Todesursachen. Aus diesem Grunde gibt es zahlreiche Bemühungen, Therapien zu entwickeln, um Krebserkrankungen zu behandeln. Viele dieser Behandlungsmethoden sind jedoch nur bedingt erfolgreich. Zum einen kann ein sich bereits im Körper etablierter Tumor in einem Organismus mit den bekannten Mitteln nur sehr schwer bekämpft werden, zum anderen zeigen die verwendeten Antitumormittel im Allgemeinen eine Vielzahl von unerwünschten Nebenwirkungen, die insbesondere bei den zu verabreichenden Dosismengen der Mittel Beschränkungen auferlegen.

Neben den Ansätzen zur Gentherapie zur Behandlung von Krebserkrankungen werden seit Jahren ausgewählte Metalle bzw. Metallverbindungen, wie zum Beispiel Vanadium, Molybdän, Gold und insbesondere auch Platin, zur Behandlung von verschiedenen Tumorerkrankungen eingesetzt. Viele dieser eingesetzten Verbindungen, wie zum Beispiel die Platin-Verbindungen, haben neben den allgemeinen Nebenwirkungen ein hohes toxisches Potential, insbesondere sind diese Verbindungen nephrotoxisch. Weiterhin zeigen vor allem Platin-Verbindungen, wie zum Beispiel die cis-Platin-Verbindungen, spezifische unerwünschte Nebenwirkungen, wie zum Beispiel starke Durchfälle, strenges Erbrechen, Verlust der Körper- insbesondere der Kopfbehaarung. Außerdem wird die Aktivität - vor allem die immunologische Aktivität - des Knochenmarks unterdrückt.

Dies führt in der Summe dazu, dass die Patienten trotz der für sie bedrohlichen Erkrankungen diese Art der Therapie vorzeitig abbrechen und andere Therapieformen zur Behandlung ihres pathogenen Zustandes wählen.

Bei den Krebstherapien zeigt sich insgesamt, dass zu behandelnde Patienten gegenüber oral zu verabreichenden Mitteln aufgeschlossen sind. So wird beispielsweise eine orale Chemotherapie von den Patienten aufgrund der Tatsache, dass sie ihr normales Leben weitestgehend fortführen können und diese Behandlungen relativ wenig Nebenwirkungen zeigen, sehr gut akzeptiert. Im Jahr 2001 waren zirka 25 % bis 30 % der in der Onkologie verwendeten Medikationen orale Formulierungen. Diese oralen Formulierungen eignen sich bedingt, um verschiedenste Krankheitsmechanismen zu behandeln. Bekannte Beispiele für orale Antitumortherapien sind zum Beispiel die Gabe von zytotoxischen Agenzien, Anti-Angiogeneseprodukten, Mitteln, die die Zellzyklusmechanismen modifizieren, Inhibitoren der Signaltransduktion und Hormonsuppressionsmittel.

Die gute Akzeptanz der oralen Chemotherapeutika führte dazu, dass es im Stand der Technik mehrere Versuche gab, pharmazeutische, oral zu applizierende Mittel bereitzustellen, die eine gute Antitumoraktivität kombiniert mit einer geringen Toxizität zeigen. Es gab daher mehrere Versuche, effektiv wirkende pharmazeutische Mittel mit geringen Nebenwirkungen bereitzustellen, die auf Molybdän-, Vanadium-, Gold- und insbesondere Platinkomponenten beruhen.

Im Stand der Technik sind insbesondere mehrere Oxoplatin-Verbindungen bekannt. So offenbart Presnov, M. A. et al in Neoplasma (1985) cis-Diammonium-cis-dichloro-transdihydroxyplatinum(IV)(Oxoplatin) zur Behandlung von Tumoren, wobei eine Salzlösung als pharmakologischer Träger für Oxoplatin fungiert. Diese Verbindungen sind jedoch instabil, da der pharmakologische Träger (Salzionen) die offenbarten Oxoplatin-Verbindungen in ihrer Stabilität negativ beeinflusst. Brandon, R J et al, Journal of Medical Chemistry (1984), beschreiben die antitumorale Wirkung einer Gruppe von Derivaten des cis-Diammonium-cis-dichloro-trans-dihydroxyplatinum(IV) sowie Injektionslösungen, die diese enthalten, zur Behandlung von Tumorerkrankungen, wobei Wasser das Trägermittel für Oxoplatin ist. Die Autoren offenbaren, dass eine Gruppe von Pt(IV)-Komplexen, welche durch die Oxidation von cis-Dichlorodiammoniumplatin(II) oder seinem cis-hydroxo Analogen gewonnen wurden, durch Infrarot- und Pt-NMR-Spektroskopie synthetisiert und gekennzeichnet wurden. Die zytotoxische Aktivität der Platin(IV)-Verbindungen ist nach Ansicht der Autoren sehr wahrscheinlich entweder auf ihre Anfälligkeit für in vivo Reduktion auf aktive Pt(II)-Arten zurückzuführen oder möglicherweise auf ihre Fähigkeit, ohne Reduktion mit wichtigen zellulären Bestandteilen wie z. B. der DNA zu interagieren. Es stellte sich heraus, dass die Pt(IV) Verbindungen als Antitumorwirkstoffe weitaus weniger aktiv gegen L-1210 Leukämie waren als die Verbindung cis-Dichlorodiammoniumplatin(II). Der eingesetzte wässrige Träger kann jedoch eine ex-vivo Umwandlung von Platin(IV)- in Platin(II)-Verbindungen nicht verhindern, so dass weniger wirksame Platin(IV)/Pla-tin(II)-Gemische appliziert wurden. Aref' eva A K et al, Voprosy Onkolgii.USSR (1990), offenbaren Oxoplatin-Verbindungen und ihre Wirkung gegen Krebs. Es wird diskutiert, nierentoxische Probleme durch die Gabe von Diuretika zu minimieren. Nachteiligerweise führt die Gabe von Oxoplatin-Verbindungen und Diuretika zu Brechdurchfall, so dass eine Gesamtschwächung des Organismus dergestalt eintritt, dass derartige Verbindungen in der Therapie, insbesondere in der Klinik, leider nicht mit Erfolg einsetzbar sind. Es lässt sich so nicht einmal sicher feststellen, ob das durch Oxoplatin induzierte Nierenversagen durch die Gabe von Diuretika erfolgreich abgemildert werden kann. Kelland et al, Cancer Research (1994) beschreiben u. a. cis-Platin- und trans-Platin-Komplexe und ihre Antitumorwirksamkeit in vitro und in vivo. Die Platin-Verbindungen werden in 0,9%ige Natriumchloridlösung gelöst, um sie auf ihre zytotoxische Wirkung auf Tumorzellen zu untersuchen. Weiterhin wird im Stand der Technik Arachidöl als Träger offenbart. Dieser Träger verursacht bei einem Teil der Patienten jedoch starke allergische Reaktionen. Die RU-C-2 086 261 (1997) offenbart peroral verabreichte pharmazeutische Präparate, die cis-Diammonium-cis-dichloro-trans-dihydroxyplatinum(IV) zur Behandlung von Tumorerkrankungen enthalten, wobei als Träger Natriumbicarbonat und Natriumalginat verwendet werden. Behandelt werden insbesondere Leukämie, das Adenokarzinom, das Melanom, der Zervikalkrebs, das Sarkom und das Hepatom. Die so offenbarten pharmazeutischen Träger führen allerdings in Verbindung mit den Platin-Derivaten zu einer Veränderung der lokalen Säure/Base Situation in dem Organismus, wodurch die Bioverfügbarkeit und die Biotransformation von Oxoplatin so verändert werden, dass die Tumoren nicht therapiert werden können. Der genannte Stand der Technik offenbart keine Verbindungen, die in geringen Dosen ohne Nebenwirkungen wirksam sind und die insbesondere durch den pharmazeutischen Träger eine Stabilität des Oxoplatins dergestalt garantieren, dass das Oxoplatin in der erforderlichen Konzentration bioverfügbar ist und innerhalb des Körpers biotransformiert.

Bisher ist es nicht gelungen, oral zu verwendende pharmazeutische Mittel, insbesondere chemotherapeutisch anwendbare Verbindungen, auf Basis von Platin-Verbindungen bereitzustellen, die eine geringe toxische, insbesondere nephrotoxische Aktivität aufweisen. Weiterhin konnten bisher noch keine für den Patienten akzeptierbaren Platin-umfassende pharmazeutischen Mittel entwickelt werden, die beispielsweise als Creme oder Salbe, insbesondere für die Behandlung von Tumorerkrankungen der Haut, angewendet werden können.

Aufgabe der Erfindung war es daher, Platin-umfassende pharmazeutische Mittel, insbesondere chemotherapeutische Mittel, bereitzustellen, die eine geringe Toxizität, insbesondere Nephrotoxizität, aufweisen und gut als orale Mittel bzw. als Creme oder als Salbe in der Tumortherapie eingesetzt werden können.

Die Erfindung löst diese erfindungsgemäße Aufgabe durch ein pharmazeutisches Mittel, welches cis-Diammoniumdichloro-transdihydroxoplatin (IV) und/oder dessen Salze umfasst. Überraschenderweise wurde gefunden, dass cis-Diammoniumdichlorotransdihydroxoplatin (IV) bzw. cis-Oxoplatin oder Oxoplatin umfassende pharmazeutische Verbindungen im Vergleich zu beispielsweise cis-Platin-Verbindungen nur eine geringe toxische Aktivität aufweisen, insbesondere sind sie kaum oder gar nicht nephrotoxisch. Cis-Oxoplatin-umfassende pharmazeutische Mittel haben auch eine geringere Halbwertszeit im Körper als vergleichbare cis-Platin-Verbindungen, das heißt, wichtige Stoffwechselorgane - wie beispielsweise die Leber oder die Niere - werden durch die erfindungsgemäßen Verbindungen weniger belastet. So war es zum Beispiel überraschend, dass 20 Tage nach einer Injektion von cis-Oxoplatin die Nieren weitgehend frei von dieser Verbindung sind, wohingegen nach einer Vergleichsinjektion mit cis-Platin der Level an Platin in der Niere ungefähr so hoch war wie nach einer Stunde nach der Injektion. Besonders bevorzugt ist der Einsatz der Salze, insbesondere der Kalium-, Natrium-, Magnesium- oder Calcium-Salze, wobei die Anionen zum Beispiel Chloride, Sulfate, Phosphate, Nitrate oder Carbonate sein können. Dem Fachmann sind weitere Elemente bekannt, die Salze bilden können, zum Beispiel die Elemente der 1 - 5 Hauptgruppe des Periodensystems der Elemente, sowie die Elemente der 1 - 8 Nebengruppe.

Weiterhin zeigen die erfindungsgemäßen Verbindungen Tendenzen der Biotransformation, das heißt, zunächst wird beispielsweise oral oder in Form einer Injektion ein pharmazeutisches, insbesondere chemotherapeutisches Mittel umfassend cis-Diammoniumdichlorotransdihydroxoplatin (IV) aufgenommen, welches in bestimmten Dosen effizient gegen ausgewählte Tumoren wirkt, ohne erhebliche toxische Nebenwirkungen - insbesondere im Vergleich zu cis-Platin-Verbindungen - zu zeigen. Nach einer gewissen Zeitspanne kann diese Platin(IV)-Verbindung durch Prozesse innerhalb des Organismus in eine Platin(II)-Verbindung umgewandelt werden, die wiederum spezifisch gegen bestimmte Tumoren wirkt. Platin(IV)- und Platin(II)-Verbindungen können vorteilhafterweise eine unterschiedliche Spezifität in ihrer antitumoralen Wirksamkeit aufweisen.

Insbesondere chemotherapeutische Mittel, die Salze des cis-Diammoniumdichlorotransdihydroxoplatin (IV) umfassen, sind sehr gut löslich und daher sehr gut bioverfügbar. Aus diesem Grunde können sie in einer geringeren Konzentration eingesetzt werden und haben dennoch eine höhere Wirksamkeit und weniger Nebenwirkungen als die vergleichbare Base. Diese gute Löslichkeit führt auch dazu, dass sich die Salze für Kombinationen mit anderen aktiven Wirkstoffen, Vitaminen oder anderen Antitumormitteln gut eignen. Vor allem sind diese Salze gut in solchen sauren Milieus lösbar, wie sie beispielsweise im humanen oder tierischen Magen vorherrschen. Im Gegensatz zu den entsprechenden Basen lösen sich die Salze, sobald sie mit Magensäure in Kontakt kommen, um ein Vielfaches besser auf und können daher beispielsweise im Magen bei Magentumoren sofort ihre Wirkung entfalten. Die erfindungsgemäßen Verbindungen sind auch deshalb vorteilhafterweise insbesondere für die Magen-Darm-Passage geeignet, da sie wenig Addukte bilden. Diese verringerte Adduktbildung ist jedoch nicht nur auf die Regionen des Verdauungstraktes beschränkt, sondern bezieht sich auch auf das Verhalten der erfindungsgemäßen Verbindungen in Niere und Leber. Unter Addukten im Sinne der Erfindung sind schädliche, nebenwirkungsreiche Produkte zu verstehen, die sich durch den Ab- und Umbau der erfindungsgemäßen Verbindungen in einem Organismus ergeben können.

Die erfindungsgemäßen pharmazeutischen Mittel sind in der Lage, an eine DNA direkt zu binden. Die cis-Oxoplatin-Verbindungen gemäß der Erfindung weisen eine oktaedrische Form auf. Cis-Oxoplatin im Sinne der Erfindung kann daher sowohl Intra- als auch Inter-Strang-DNA-Komplexe bilden. Aufgrund der spezifischen Struktur von cis-Oxoplatin im Gegensatz zu cis-Platin gehen die erfindungsgemäßen Verbindungen mehrere Bindungen mit DNA-Strängen ein. Durch die Inter-Strang-Crosslinkerkomplexe und die Intra-Strang-Crosslinkerkomplexe zeigen die erfindungsgemäßen Verbindungen spezifische zytostatische vorteilhafte Effekte in der Antitumortherapie. Die DNA-Addukte der erfindungsgemäßen Verbindungen zeigen eine höhere Ladung des Platinzentralatoms und weisen weiterhin zwei weitere zusätzliche Liganden, die an dieses Zentrum gebunden sind, auf. Durch die Oktaederform der erfindungsgemäßen Platin(IV)-Komplexe ist eine sehr spezifische, relativ langsame DNA-Bindung möglich, die eine effektivere Wirkung zeigen kann als beispielsweise die Bindung von cis-Platin mit der DNA. Vorteilhaft ist es weiterhin, dass cis-Oxoplatin in vergleichbaren Konzentrationen Proteasen wie beispielsweise Trypsin oder α-Chemotrypsin im Gegensatz zu cis-Platin nicht inhibiert. Die Wirksamkeit und Effektivität der erfindungsgemäßen cis-Oxoplatin (IV)-umfassenden pharmazeutischen Mittel ist weitestgehend unabhängig von der Form der Applikation dieser Mittel. Die Mittel können peroral, oral, rektal, subkutan, intramuskulär, intravenös und intraperitoneal verabreicht werden. Zusätzliche Vorteile der erfindungsgemäßen Verbindungen gegenüber den bekannten cis-Platin-Verbindungen sind weiterhin, dass der therapeutische Effekt der erfindungsgemäßen Verbindungen länger anhält, weiterhin sind die erfindungsgemäßen Verbindungen in verschiedenen Stadien des Tumorwachstums hoch effektiv und cis-Oxoplatin-Verbindungen zeigen in der Therapie einen länger anhaltenden positiven Effekt als vergleichbare cis-Platin-Verbindungen. Diese Eigenschaften in der Löslichkeit, der Pharmakogenetik, in der Bioverfügbarkeit sowie in dem Abbau und der Adsorption innerhalb des Körpers gestatten mit den erfindungsgemäßen Mitteln eine effektivere Behandlung von Tumorerkrankungen als mit bekannten Platinumfassenden pharmazeutischen Mitteln.

In einer bevorzugten Ausführungsform der Erfindung ist das erfindungsgemäße pharmazeutische Mittel ein chemotherapeutisches Mittel, welches in der Tumorprophylaxe und/oder -therapie angewendet wird. Tumorprophylaxe im Sinne der Erfindung heißt entweder die Verhinderung der Ausbildung eines Tumors bzw. die Inhibierung des Wachstums bzw. das Stoppen des Wachstums von einzelnen tumorartigen Geweben sowie die Verhinderung bzw. Verminderung der Metastasierung von Tumoren, die Verhinderung oder die Reduzierung der Invasion von einzelnen Tumorzellen in umgebendes Gewebe sowie die Unterdrückung bzw. Inhibierung der Angiogenese in Verbindung mit der Tumorentwicklung. Das chemotherapeutische Mittel kann daher zur Verhinderung von Tumoren eingesetzt werden, beispielsweise wenn schon kleinere Tumoren vorhanden sind; aber auch zur Prophylaxe, um die Tumorentwicklung frühzeitig zu unterbinden.

Werden die erfindungsgemäßen Mittel zur Prophylaxe eingesetzt, können sie als Impfstoff verwendet werden. Dem Fachmann sind verschiedene Möglichkeiten der Formulierung und Generierung - beispielsweise bei der Auswahl von Trägern oder Lösungsmitteln - von Impfstoffen bekannt.

Bevorzugt werden die chemotherapeutischen Mittel als Kapsel, Tablette, Creme, Salbe, Zäpfchen und/oder Infusionslösung eingesetzt. Die erfindungsgemäßen chemotherapeutischen Mittel können sowohl Krebszellen als auch andere Krankheitserreger, wie zum Beispiel Parasiten, Viren, Bakterien und andere, in ihrer Ausbreitung und ihrem Wachstum hemmen. Beispielsweise ist es möglich, dass durch Hepatitisviren verursachte Lebertumoren durch die erfindungsgemäßen chemotherapeutischen Mittel behandelt werden, indem beispielsweise das oral gegebene Mittel sowohl auf die Hepatitisviren als auch auf die durch sie induzierten Krebszellen wirkt.

Eine Kapsel oder eine Tablette im Sinne der Erfindung sind Applikations- bzw. Darreichungsformen, die insbesondere eine orale Aufnahme durch den Patienten ermöglichen. Die Tabletten und Kapseln im Sinne der Erfindung schließen andere oral aufnehmbare pharmazeutische Mittel mit ein, wie beispielsweise Dragees, Pillen und Zäpfchen, aber auch Tropfen, Sirupe oder Säfte.

Zäpfchen im Sinne der Erfindung sind alle die Darreichungsformen, die beispielsweise rektal oder vaginal durch den Organismus aufgenommen werden können.

Tabletten, Dragees, Kapseln, Pillen, Pulver und Zäpfchen sind im Wesentlichen feste Arzneiformen. Diese festen Arzneiformen können ineinander übergehen. So ist es beispielsweise möglich, dass die Tablette bereits mit der oralen Aufnahme auf der Zunge in ein Pulver zerfällt oder eine in ihr befindliche Lösung, wie beispielsweise einen Sirup, freigibt. Neben dem eigentlichen Wirkstoff umfassen die erfindungsgemäßen Tabletten auch Hilfsstoffe bzw. Bindemittel. Bei den Hilfsstoffen kann es sich beispielsweise um Stärke, Mannit, Milchzucker, Zucker, Alkohole oder Kalziumsulfat handeln. Bei den Bindemitteln kann es sich beispielsweise um Cellulose oder Mannitol handeln.

Die Tabletten im Sinne der Erfindung können beispielsweise Filmtabletten sein. Filmtabletten sind mit Lack überzogene Tabletten. Bei Filmtabletten und Dragees werden Überzüge schichtweise in Form von Lösungen aufgetragen und danach angetrocknet. Filmtabletten werden insbesondere dann angewendet, wenn der erfindungsgemäße Wirkstoff, nämlich das cis-Diammoniumdichlorotransdihydroxoplatin (IV), erst im Dünndarm in hoher Konzentration zur Wirkung kommen soll. Weiterhin ist es möglich, dass die Tabletten Manteltabletten sind. Bei Manteltabletten werden auf den Kern in trockener Form eine oder mehrere Überzüge mit Druck aufgepresst. Hierdurch wird ermöglicht, dass in den Manteltabletten eine Kombination von miteinander unverträglichen Wirkstoffen in einer Zubereitung - in Kern und Mantel - untergebracht werden kann. Dieses Prinzip gilt selbstverständlich auch für Schichttabletten, die cis-Oxoplatin umfassen. Hierbei handelt es sich um erfindungsgemäße Tabletten, die Schichten mit unterschiedlichem Freigabe- und Lösungsverhalten umfassen, die aufeinander gepresst werden. Weiterhin können die Manteltabletten in Form von Depottabletten und/oder Depotdragees verabreicht werden. Mit dieser Applikationsform können insbesondere erforderliche Arzneistoffkonzentrationen im Organismus über einen längeren Zeitraum aufrechterhalten werden. Dem Fachmann ist die Herstellung von Filmtabletten und Manteltabletten bekannt. Weitere oral aufnehmbare Applikationsformen im Sinne der Erfindung sind Kapseln, Pellets, Pulver und Puder.

Bei den Kapseln kann es sich beispielsweise um Hartgelatinekapseln handeln, die Einzeldosen der cis-Oxoplatin-Verbindungen in einer Hülle aus Gelatine enthalten. Es ist jedoch auch möglich, Weichgelatinekapseln zu verwenden, die die erfindungsgemäßen Wirkstoffe in flüssiger Form, beispielsweise in Form einer Lösung oder Suspension aufnehmen. Sie können neben der oralen auch für die rektale und vaginale Applikation verwendet werden. Bevorzugt ist weiterhin die Verwendung von Hartgelatinesteckkapseln, bei denen zirka 300 Pellets als Wirkstoffgranulatkügelchen eingefüllt sind. Vorteilhafterweise können auf diesem Wege schwer resorbierbare oder säurelabile Substanzen oder Mischungen als Prodrug verabreicht werden. Die kleinen Pellets werden vorteilhafterweise nach Auflösung der sie umgebenden Hartgelatinekapseln im Magen gleichmäßig in den Darm transportiert. Damit sind eine konstante Resorption und eine konstante Konzentration an cis-Diammoniumdichlorotransdihydroxoplatin (IV) garantiert. Die Wirkstofffreisetzung und damit der Ort und der Zeitverlauf der Resorption können durch geeignete Herstellungsverfahren der oralen Mittel gesteuert werden, die dem Fachmann bekannt sind. Die oral zu verwendenden chemotherapeutischen Mittel können mit unterschiedlichen Filmen, wie zum Beispiel aus Wachs, überzogen werden, sofern gewünscht wird, dass sich die cis-Oxoplatin-Verbindungen erst im Darm freisetzen. Dies kann beispielsweise vorteilhaft bei verschiedenen Formen des Darmkrebses sein.

Neben den festen Arzneiformen, wie insbesondere Kapseln und Tabletten sowie Zäpfchen, können auch nicht feste Arzneiformen bevorzugt sein. Hierbei handelt es sich beispielsweise um Salben, Cremes und Pasten, die zum Beispiel äußerlich auf die Haut aufgetragen werden können. Dies ist insbesondere dann vorteilhaft, wenn bestimmte Wirkstoffe nicht explizit in die Blutbahn gelangen sollen; das heißt, wenn es nicht gewünscht ist, dass diese systemisch absorbiert werden. Dies kann beispielsweise bei verschiedenen Formen des Hautkrebses vorteilhaft sein. Selbstverständlich ist es aber auch möglich, dass die Salben so zubereitet werden, dass die Wirkstoffe auch das unter der Haut liegende Gewebe erreichen und teilweise in das Blutgefäßsystem gelangen; hierbei handelt es sich um die dem Fachmann bekannten Resorptionssalben. Um an einen Wirkort unterhalb der oberen Hautschichten zu gelangen, wie es beispielsweise bei der Behandlung von bestimmten Melanomen erforderlich sein kann, ist es vorteilhaft, wenn der Wirkstoff cis-Oxoplatin die Zubereitungsform verlassen kann und durch die Haut hindurchtritt. Er muss diese insbesondere dann durchdringen können, wenn neben der topischen Wirkung auch eine systemische beabsichtigt ist. Hierfür können beispielsweise Suppositorien und transdermale therapeutische Systeme, wie zum Beispiel Nitratpflaster, eingesetzt werden. Dem Fachmann ist bekannt, wie er derartige Systeme und Darreichungsformen bereitstellen kann. So ist zum Beispiel die Tendenz zum Verlassen des Wirkstoffes aus einem Träger umso größer, je stärker sich die Lipophilie von dem Träger und dem Wirkstoff unterscheidet.

Die erfindungsgemäßen Salben bestehen zum Beispiel aus einer lipophilen Grundlage wie zum Beispiel Paraffinöl, Vaseline und Wollfett und können zirka 10 % Pulver wie Zinkoxid, Titanoxid, Stärke oder ein anderes Pulvergemisch enthalten. Bei hydrophoben Salben im Sinne der Erfindung ist die äußere Phase lipophil, das heißt, diese Salben stellen eine Emulsion von Wasser in Fett dar.

Pasten im Sinne der Erfindung sind insbesondere Fettsalben mit einem Anteil von mindestens 10 % pulverförmigen Bestandteilen.

Cremes im Sinne der Erfindung sind Zubereitungen, die aus einer lipophilen und einer hydrophilen Phase bestehen. Bei hydrophilen Cremes ist die äußere Phase die wässrige, sie entsteht beispielsweise mit Hilfe von Emulgatoren als Emulsion eines Fettes in Wasser.

Gele umfassen neben dem Wirkstoff auch Gelbildner, wie zum Beispiel Gelatine, Methylcellulose und/oder Polyethylenglykol.

Bei den Zäpfchen oder Suppositorien sind torpedoartig geformte Darreichungsformen bevorzugt, bei denen in eine Grundmasse, meist Neutralfette, der Wirkstoff cis-Oxoplatin gleichmäßig verteilt ist. Sie sind beispielsweise zum Einführen in den Enddarm oder in die Vagina bestimmt, um dort durch Schmelzen oder Auflösen die Wirkstoffe freizusetzen. Die Zäpfchen werden bevorzugt verwendet, um eine lokale Wirkung auszuüben oder um Substanzen rektal oder vaginal zu resorbieren, wie beispielsweise bei einem Tumor in der Region der Vagina oder einem Dickdarmkrebs. Dies ist insbesondere dann bevorzugt, wenn der Patient aufgrund der Nebenwirkungen von anderen Antitumormitteln zu ständigem Erbrechen neigt oder wenn eine Leberpassage unmittelbar nach der Resorption vermieden werden soll oder auch um einen schnellen Abbau zu verhindern. Mit Vorteil wird der größte Teil der rektal resorbierten cis-Oxoplatin-Verbindungen unter Umgehung der Leberpassage direkt dem großen Blutkreislauf zugeführt. Neben den Scheidenzäpfchen können auch Vaginalkugeln (Ovula) bevorzugt sein.

Bei den flüssigen Darreichungsformen kann es sich im Sinne der Erfindung bevorzugt um Infusionslösungen, aber auch um Sirupe oder Säfte handeln. Beispielsweise können derartige Lösungen zum Gurgeln oder Spülen angeboten werden, sofern ein Zungentumor oder ein Tumor der oberen Halsregion behandelt werden soll. Insbesondere die erfindungsgemäßen Sirupe können süß schmeckende Zucker- und Zuckeraustauschstoffe enthalten.

Die erfindungsgemäßen Injektions- und Infusionslösungen sind im Wesentlichen frei von Infektionserregern oder von Schwebstoffen. Die Injektions- oder Infusionslösungen werden nach der Herstellung luftdicht abgeschlossen und in Behältnissen aus Glas oder Kunststoff aufbewahrt, die farblos sein können oder wegen eventueller Lichtempfindlichkeit bevorzugt braun eingefärbt sind. Ölige Formulierungen werden insbesondere dann eingesetzt, wenn subkutane oder intramuskuläre Depots im Organismus erzeugt werden sollen.

In einer bevorzugten Ausführungsform der Erfindung umfassen die erfindungsgemäßen Kapseln neben den cis-Oxoplatin-Verbindungen Siliciumdioxid und Mannitol bzw. Siliciumdioxid und Magnesiumstearat oder ein Gemisch aus lyophilisierten eingekapselten oder gebundenen cis-Oxoplatin an bzw. im Vehikel wie beispielsweise Siosomen, Liposomen und/oder Nanosomen. Durch die Auswahl der genannten pharmazeutischen Hilfsstoffe und Träger ist es vorteilhafterweise möglich zu wählen, ob der Wirkstoff cis-Oxoplatin schnell oder langsam nach Aufnahme in den Organismus freigesetzt werden soll.

Die erfindungsgemäßen Kapseln, insbesondere solche, die den Wirkstoff schnell freigeben, umfassen cis-Oxoplatin : Siliciumdioxid : Mannitol oder Magnesiumstearat bevorzugt im Verhältnis von 0,1 bis 10 : 0,1 bis 10 : 0,1 bis 10, besonders bevorzugt von 0, 5 bis 5 : 0, 5 bis 5 : 0, 5 bis 5 und ganz besonders bevorzugt von 0,7 bis 2 : 0,7 bis 2 : 0,7 bis 2, insbesondere im Verhältnis 1 : 1 : 1. So kann beispielsweise die Kapsel im Sinne der Erfindung, 50 mg Siliciumdioxid und 50 mg Mannitol 50 mg Oxoplatin oder 50 mg Magnesiumstearat umfassen. Je nach Herstellungsverfahren eignen sich derartige Kapseln auch zur langsamen Freisetzung der Wirkstoffe. Dem Fachmann ist die Wahl derartiger Herstellungsverfahren bekannt. Selbstverständlich ist es auch möglich, dass die Kapseln Lipidvehikel wie Siosomen, Liposomen, Nanokapseln oder Nanosomen umfassen. Durch die Auswahl der entsprechenden Siosomen, Liposomen und Nanosomen kann eingestellt werden, ob das cis-Oxoplatin schnell oder langsam freigesetzt werden soll. Dem Fachmann ist bekannt, dass er die Freisetzung wie auch die Resorption oder Aufnahme bzw. die Verteilung, den Abbau und die Ausscheidung der entsprechenden Wirksubstanzen, meist den gesamten Komplex der Pharmakokinetik und der Pharmakodynamik der erfindungsgemäßen chemotherapeutischen Mittel beeinflussen kann.

In einer bevorzugten Ausführungsform der Erfindung umfasst die Tablette neben cis-Oxoplatin Lactose, Maisstärke, Poly(o-Carboxymethyl)stärke-Natriumsalz, Calciumhydrogenphosphat 2H₂O, Cellulosepulver und Magnesiumstearat oder Siliciumdioxid und Magnesiumstearat.

In einer besonderen Ausführungsform beträgt das Verhältnis von cis-Oxoplatin : Lactose : Maisstärke : Poly(o-Carboxymethyl)stärke-Natriumsalz . Calciumhydrogenphosphat 2H₂O : Cellulosepulver : Magnesiumstearat 10 bis 500 : 20 bis 150 : 1 bis 10 : 1 bis 10 : 1 bis 10 : 1 bis 10 : 0, 1 bis 7; besonders bevorzugt 20 bis 200 : 40 bis 100 : 2 bis 8 : 2 bis 8 : 2 bis 8 : 2 bis 8 : 0,5 bis 5 und ganz besonders bevorzugt 50 bis 150 : 60 bis 90 : 3 bis 7 : 3 bis 7 : 3 bis 7 : 3 bis 7 : 0,7 bis 1, insbesondere 100 : 79 : 5 : 5 : 5 : 5 : 1.

Eine vorteilhafte Tablette enthält demgemäß zum Beispiel 50 mg cis-Oxoplatin, 39,5 mg Lactose, 2,5 mg Maisstärke, 2,5 mg Poly(o-Carboxymethyl)stärke-Natriumsalz, 2,5 mg Calciumhydrogenphosphat 2H₂O, 2,5 mg Cellulosepulver und 0,5 mg Magnesiumstearat. Bevorzugt eignen sich derartige Tabletten zur schnellen Freisetzung des Wirkstoffes. In einer weiteren vorteilhaften Tablette im Sinne der Erfindung werden beispielsweise statt 39,5 mg Lactose 39 mg Lactose eingesetzt und statt 2,5 mg Maisstärke 2 mg Maisstärke pro Tablette.

Es kann jedoch auch bevorzugt sein, Tabletten einzusetzen, die den Wirkstoff sehr langsam freisetzen. Derartige Tabletten umfassen cis-Oxoplatin : Siliciumdioxid : Magnesiumstearat bevorzugt im Verhältnis von 0,1 bis 10 : 0,1 bis 10 : 0,1 bis 10, besonders bevorzugt 0,5 bis 5 : 0,5 bis 5 : 0,5 bis 5 und ganz besonders bevorzugt 0,7 bis 2 : 0,7 bis 2 : 0,7 bis 2, insbesondere 1 : 1 : 1. Demgemäß kann eine Tablette, welche ihre Wirkstoffe langsam freigibt, 50 mg cis-Oxoplatin, 50 mg Siliciumdioxid und 50 mg Magnesiumstearat umfassen.

Bevorzugt ist es weiterhin, eine Creme zu verwenden, die neben cis-Oxoplatin Benzylalkohol, Cestylstearylalkohol, Macrogolstearat 1000, Isopropylpalmitat, Glycerol, Sorbitol-Lösung, bevorzugt 70 %, ganz besonders bevorzugt nicht kristallisierend, und gereinigtes Wasser umfasst.

In einer bevorzugten Ausführungsvariante bei der Ausgestaltung einer erfindungsgemäßen Creme beträgt das Verhältnis von cis-Oxoplatin : Benzylalkohol : Cestylstearylalkohol : Macrogolstearat 1000 : Isopropylpalmitat : Glycerol : Sorbitol-Lösung 70 % : Wasser bevorzugt 0,2 bis 8 : 0,1 bis 7 : 1 bis 10 : 0,1 bis 7 : 0,1 bis 7 : 0,2 bis 8 : 0,2 bis 8 : 20 bis 60, besonders bevorzugt 0,4 bis 4 : 0,2 bis 3 : 2 bis 9 : 0,2 bis 3 : 0,2 bis 3 : 0,4 bis 4 : 0,4 bis 4 : 25 bis 45 und ganz besonders bevorzugt von 0,7 bis 3 : 0,5 bis 1,5 : 4 bis 6 : 0,5 bis 1,5 : 0,5 bis 1,5 : 0,7 bis 3 : 0,7 bis 3 : 30 bis 40, insbesondere 2,5 : 1 : 5 : 1,25 : 1 : 2 : 2,5 : 2,5 : 35,25. Demgemäß ist eine bevorzugte erfindungsgemäße Creme beispielsweise zusammengesetzt aus 50 mg cis-Oxoplatin, 20 mg Benzylalkohol, 100 mg Cestylstearylalkohol, 25 mg Macrogolstearat 1000, 20 mg Isopropylpalmitat, 40 mg Glycerol, 50 mg Sorbitol und 205 mg gereinigtem Wasser.

Im Falle einer Verwendung des chemotherapeutischen Mittels als Salbe ist es bevorzugt, eine Salbe einzusetzen, die neben cis-Oxoplatin weiße Vaseline, Cestylstearylalkohol, Macrogolstearat 1000 und Propylenglykol umfasst.

In einer bevorzugten Ausführungsform der Erfindung umfasst die Salbe die einzelnen Bestandteile im Verhältnis von cis-Oxoplatin : Propylenglykol : Macrogolstearat 1000 : Cestylstearylalkohol : weiße Vaseline im Verhältnis von 2 bis 20 : 5 bis 40 : 0,1 bis 7 : 1 bis 10 : 25 bis 400, besonders bevorzugt im Verhältnis von 5 bis 12 : 10 bis 30 : 0,2 bis 3 : 2 bis 9 : 50 bis 250 und ganz besonders bevorzugt im Verhältnis von 6 bis 10 : 15 bis 25 : 0,5 bis 1,5 : 3 bis 6 : 100 bis 200 und insbesondere im Verhältnis von 9,1 : 22 : 1 : 4 : 155. Demgemäß kann die erfindungsgemäße Salbe beispielsweise 50 mg cis-Oxoplatin, 120 mg Propylenglykol, 5,5 mg Macrogolstearat 1000, 22 mg Cestylstearylalkohol und 851,5 mg weiße Vaseline umfassen.

Bei einer Verwendung als Gel, insbesondere als Formgel, zur topischen Anwendung ist es bevorzugt, dass das Gel Hydroxyethylcellulose, Chloroaerosol, Natriumhydroxid, Natriumhydrogenphosphatdihydrat und gereinigtes Wasser umfasst.

In einer bevorzugten Ausführungsform der Erfindung umfasst das Formgel cis-Oxoplatin : Hydroxyethylcellulose : Chloroaerosol : Natriumhydroxid : Natriumhydrogenphosphatdihydrat : gereinigtem Wasser im Verhältnis von 2 bis 20 : 100 bis 600 : 5 bis 40 : 0,1 bis 7 : 20 bis 60 : 3000 bis 50000, besonders bevorzugt im Verhältnis von 4 bis 18 : 200 bis 500 : 10 bis 30 : 0,2 bis 3 : 25 bis 45 : 5000 bis 35000 und ganz besonders bevorzugt im Verhältnis von 6 bis 12 : 300 bis 400 : 15 bis 25 : 0,5 bis 1,5 : 30 bis 40 : 10000 bis 30000 und insbesondere im Verhältnis von 10 : 360 : 20 : 1 : 34 : 19569. So kann beispielsweise ein Formgel 0,05 g cis-Oxoplatin, 1,8 g Hydroxyethylcellulose, 0,1 g Chloroaerosol, 0,005 g Natriumhydroxid, 0,17 g Natriumhydrogenphosphatdihydrat und 97,875 g gereinigtes Wasser umfassen, so dass 100 g des bevorzugten erfindungsgemäßen Gels bereitgestellt werden können.

In einer weiteren Ausführungsform der Erfindung ist es bevorzugt, eine Zäpfchenform, insbesondere zur analen oder vaginalen Applikation, zu verwenden, die hochdisperses Siliciumdioxid und Hartfett umfasst oder aber Lactose, Maisstärke, Adipinsäure, Natriumhydrogencarbonat; Stearinsäure, Magnesiumstearat, hochdisperses Siliciumdioxid und Polysorbat 80.

In einer bevorzugten Ausführungsform der Erfindung umfassen die Analzäpfchen cis-Oxoplatin und hochdisperses Siliciumdioxid und Hartfett im Verhältnis von 0,1 bis 10 : 0,1 bis 10 : 30 bis 300, besonders bevorzugt im Verhältnis von 0,2 bis 4 : 0,2 bis 4 : 40 bis 200 und ganz besonders bevorzugt im Verhältnis von 0,5 bis 2 : 0,5 bis 2 : 60 bis 150 und insbesondere im Verhältnis von 1 : 1 : 92,5; das heißt, eine solche Applikationsform kann beispielsweise 0,02 g cis-Oxoplatin, 0,02 g hochdisperses Siliciumdioxid und 1,85 g Hartfett umfassen. Ein weiteres bevorzugtes Zäpfchen umfasst cis-Oxoplatin : Lactose : Maisstärke : Adipinsäure : Natriumhydrogencarbonat : Stearinsäure : Magnesiumstearat : hochdispersem Siliciumdioxid : Polysorbat 80 im Verhältnis von 10 bis 100 : 700 bis 4000 : 200 bis 600 : 10 bis 1000 : 10 bis 1000 : 1 bis 100 : 1 bis 100 : 1 bis 15 : 0,1 bis 10, besonders bevorzugt im Verhältnis von 20 bis 80 : 1000 bis 3000 : 250 bis 450 : 20 bis 400 : 20 bis 400 : 2 bis 40 : 2 bis 40 : 2 bis 10 : 0,2 bis 4 und ganz besonders bevorzugt 30 bis 60 : 1500 bis 2500 : 300 bis 400 : 50 bis 200 : 50 bis 200 : 5 bis 20 : 5 bis 20 : 4 bis 8 : 0,5 bis 2 und insbesondere im Verhältnis von 40 : 2111 : 340 : 127 : 100 : 10 : 9 : 6 : 1. Demgemäß kann ein bevorzugtes Analzäpfchen 20 mg cis-Oxoplatin, 1055, 40 mg Lactose, 170 mg Maisstärke, 63,60 mg Adipinsäure, 50 mg Natriumhydrogencarbonat, 5 mg Stearinsäure, 4,5 mg Magnesiumstearat, 3 mg hochdisperses Siliciumdioxid und 0,5 mg Polysorbat 80 umfassen.

Neben der Anwendung zur analen Applikation kann selbstverständlich auch die vaginale Applikationsform bevorzugt sein, die besonders bevorzugt umfasst Lactose 1H₂O, Maisstärke, Adipinsäure, Natriumhydrogencarbonat, Stearinsäure, Magnesiumstearat, hochdisperses Siliciumdioxid und Polysorbat 80.

In einer bevorzugten Ausführungsform der Erfindung umfasst das Vaginalzäpfchen cis-Oxoplatin : Lactose 1H₂O : Maisstärke : Adipinsäure : Natriumhydrogencarbonat : Stearinsäure : Magnesiumstearat : hochdispersem Siliciumdioxid : Polysorbat 80 im Verhältnis von 10 bis 100 : 1000 bis 5000 : 300 bis 1000 : 10 bis 1000 : 10 bis 1000 : 1 bis 100 : 1 bis 100 : 1 bis 15 : 0,1 bis 7, besonders bevorzugt im Verhältnis von 20 bis 90 : 1500 bis 3500 : 400 bis 800 : 20 bis 400 : 20 bis 400 : 2 bis 40 : 2 bis 40 : 2 bis 10 : 0,2 bis 3 und ganz besonders bevorzugt im Verhältnis von 30 bis 60 : 2000 bis 3000 : 500 bis 600 : 50 bis 200 : 50 bis 200 : 5 bis 20 : 5 bis 20 : 4 bits 8 : 0,5 bis 1,5 und insbesondere im Verhältnis von 40 : 2700 : 567 : 130 : 100 : 10 : 9 : 6 : 1. Beispielsweise kann ein solches Scheidenzäpfchen daher in einer bevorzugten Ausführungsform 20 mg cis-Oxoplatin, 1350 mg Lactose 1H₂O, 170 mg Maisstärke, 65 mg Adipinsäure, 50 mg Natriumhydrogencarbonat, 5 mg Stearinsäure, 4,5 mg Magnesiumstearat, 3 mg hochdisperses Siliciumdioxid und 0,5 mg Polysorbat 80 umfassen.

Ein weiteres bevorzugtes Vaginalzäpfchen umfasst die gleichen Inhaltsstoffe bevorzugt im Verhältnis von 10 bis 1000 : 1500 bis 5000 : 300 bis 1000 : 10 bis 1000 : 10 bis 1000 : 1 bis 100 : 1 bis 100 : 1 bis 15 : 0,1 bis 7, besonders bevorzugt im Verhältnis von 20 bis 400 : 2000 bis 4000 : 400 bis 800 : 20 bis 400 : 20 bis 400 : 2 bis 40 : 2 bis 40 : 2 bis 10 : 0,2 bis 3 und ganz besonders bevorzugt im Verhältnis von 50 bis 200 : 2500 bis 3500 : 500 bis 600 : 50 bis 200 : 50 bis 200 : 5 bis 20 : 4 bis 8 : 0,5 bis 1,5 und insbesondere im Verhältnis von 100 : 2900 : 567 : 130 : 100 : 10 : 9 : 6 : 1. Demgemäß kann ein solches Zäpfchen in einer bevorzugten Ausführungsform 50 mg cis-Oxoplatin, 1450 mg Lactose 1H₂O, 170 mg Maisstärke, 65 mg Adipinsäure, 50 mg Natriumhydrogencarbonat, 5 mg Stearinsäure, 4,5 mg Magnesiumstearat, 3 mg hochdisperses Siliciumdioxid und 0,5 mg Polysorbat 80 umfassen.

Selbstverständlich kann es auch bevorzugt sein, das erfindungsgemäße chemotherapeutische Mittel in Form von Injektions- und Infusionslösungen einzusetzen. In einer bevorzugten Ausführungsform der Erfindung umfasst die Injektionslösung neben cis-Oxoplatin Benzylalkohol, Polysorbat 80, Sorbitollösung, bevorzugt 70 %, besonders bevorzugt nicht kristallisierend, und Wasser oder Mannitol und Wasser.

In einer bevorzugten Ausführungsform der Erfindung umfasst die Injektionslösung cis-Oxoplatin im Verhältnis zu Benzylalkohol : Polysorbat 80 : Sorbitollösung 70 % : Wasser im Verhältnis von 0,2 bis 8 : 1 bis 10 : 0,1 bis 7 : 100 bis 800 : 100 bis 400, besonders bevorzugt im Verhältnis von 0,4 bis 4 : 2 bis 9 : 0,2 bis 3 : 200 bis 600 : 150 bis 350 und ganz besonders bevorzugt im Verhältnis von 0,7 bis 3 : 3 bis 6 : 0,5 bis 1,5 : 250 bis 400 : 200 bis 300 und insbesondere im Verhältnis zu 2,5 : 4,5 : 1 : 325 : 250. Demgemäß kann beispielsweise eine Präparation einer 5 mg/ml Injektionslösung 5 mg cis-Oxoplatin, 9 mg Benzylalkohol, 2 mg Polysorbat 80, 650 mg Sorbitollösung 70 % und 500 mg Wasser enthalten.

Eine weitere bevorzugte Injektionslösung umfasst cis-Oxoplatin : Mannitol : Wasser im Verhältnis von 0,1 bis 7 : 5 bis 40 : 1 bis 10 und besonders bevorzugt im Verhältnis von 0,2 bis 3 : 10 bis 30 : 2 bis 9 und ganz besonders bevorzugt im Verhältnis von 0,5 bis 1,5 : 15 bis 25 : 3 bis 6 und insbesondere im Verhältnis von 1 : 20 : 4. Demgemäß kann eine Präparation einer 5 mg/ml Injektionslösung 5 mg cis-Oxoplatin, 100 mg Mannitol und 200 ml Wasser für die Injektion umfassen.

Die Erfindung betrifft auch die Herstellung der Kapseln, Tabletten, Zäpfchen, Salben, Cremes und/oder Infusionslösungen. Je nach der Wirkstofffreisetzung, beispielsweise spontan oder zeitversetzt bzw. im Magen oder im Dünndarm, können beispielsweise die oralen festen Applikationsformen bestimmte Wachsschichten - wie oben bereits ausgeführt - umfassen. Dem Fachmann ist bekannt, wie er über die Wahl bestimmter Parameter und die Auswahl bestimmter Hilfsstoffe feste orale bzw. feste Zäpfchenform bzw. Salben, Cremes und Puder bzw. flüssige Infusionslösungen bereitstellen kann. Die Herstellung der einzelnen Applikationsformen kann auch von der Art des zu behandelnden Tumors abhängen. Orale Applikationsformen, wie beispielsweise Tabletten und Kapseln, zur Behandlung von Dünndarm- oder Dickdarmkrebs werden bei der Herstellung zum Beispiel mit einer magensaftresistenten Schicht überzogen. Dem Fachmann sind weitere Applikationsformen bekannt, zum Beispiel Gel, Puder, Pulver, Tablette, Retard-Tablette, Premix, Emulsion, Aufgussformulierung, Tropfen, Konzentrat, Granulat, Sirup, Pellet, Boli, Kapsel, Aerosol, Spray und/oder Inhalat. Diese Zubereitungen können oral, subkutan, intravenös, intramuskulär, intraperitoneal, vaginal, rektal, nasal und/oder topisch eingesetzt werden.

Die Erfindung betrifft auch die Verwendung der erfindungsgemäßen pharmazeutischen Mittel zur Behandlung von Tumoren, weiterhin die Verwendung der erfindungsgemäßen Mittel zur Herstellung eines Arzneimittels zur Behandlung von Tumoren.

Die Behandlung der Tumoren im Sinne der Erfindung umfasst sowohl die prophylaktische als auch die therapeutische Behandlung des Tumors. Hierbei kann das erfindungsgemäße pharmazeutische Mittel als Vakzin nach der Etablierung eines Tumors oder als vorbeugende Impfung eingesetzt werden. Die Impfung erfolgt vorteilhafterweise so, dass es nach der Applikation in einem Organismus zur Entwicklung eines Schutzes gegen die Ausbreitung oder die weitere Bildung von Tumoren kommt. Selbstverständlich ist es auch möglich, dass die Impfung unmittelbar vor oder zeitnah nach der Manifestation eines Tumors erfolgt oder in Form einer Therapie mehrfach appliziert wird. Dem Fachmann ist bekannt, dass die Behandlung eines Tumors zu nahezu jedem Zeitpunkt auch nach der Bildung von Metastasen von Vorteil sein kann, so dass eine Impfung im Sinne der Erfindung auch eine Applikation des erfindungsgemäßen pharmazeutischen Mittels Wochen, Monate, Jahre bzw. Jahrzehnte nach der Bildung eines Tumors sein kann. Bei dem Einsatz der erfindungsgemäßen pharmazeutischen Mittel als Therapeutikum geht es insbesondere darum, die pharmazeutischen Mittel in einer solchen Menge und Applikationsform mit einem Organismus so in Kontakt zu bringen, dass ein Tumor in seinem Wachstum gehemmt wird bzw. dass die Ausbreitung des Tumors im Organismus in Form von Metastasen verhindert wird, dass die Tumorangiogenese inhibiert wird und dass die Tumorinvasion, das heißt das Eindringen von einzelnen Zellen in das Körpergewebe, verhindert oder gehemmt wird. Das In-Kontakt-Bringen erfolgt zum Beispiel oral, über Injektion, topisch, vaginal, rektal und/oder nasal.

Die an einem Gesunden im Falle der Prophylaxe bzw. an einem Patienten im Falle der Therapie zu verwendende Menge an erfindungsgemäßen Verbindungen wird formuliert und die Dosis gemäß üblicher medizinischer Praxis festgesetzt, wobei die zu behandelnde Störung, der Zustand des einzelnen Patienten, die Verabreichungsstelle, das verabreichungsverfahren und andere, den behandelnden Ärzten bekannte Faktoren berücksichtigt werden. In ähnlicher Weise hängt die Dosis der verabreichten erfindungsgemäßen Verbindungen von den Eigenschaften des Tumors ab, von der in vivo Halbwertszeit erfindungsgemäßen Verbindungen im Plasma wie auch von der Konzentration der erfindungsgemäßen Verbindungen in der Formulierung, dem Verabreichungsweg, der Stelle und der Rate der Dosierung, der klinischen Toleranz des jeweiligen Individuums (Mensch und Tier), der pathologischen Affektion des Patienten und dergleichen, wie es Ärzten bzw. anderen Fachleuten bekannt ist. Im Allgemeinen werden Dosierungen von etwa 0,1 bis 1000 mg pro Individuum und Verabreichung bevorzugt; besonders bevorzugt ist eine Dosierung von 10 bis 500 mg, ganz besonders bevorzugt von 200 bis 400 mg, insbesondere von 300 mg. Es können während einer Abfolge aufeinander folgender Verabreichungen auch unterschiedliche Dosierungen eingesetzt werden.

Die Erfindung betrifft auch einen Kit zur Therapie von Tumorerkrankungen, der die erfindungsgemäßen pharmazeutischen Mittel, insbesondere das Chemotherapeutikum, umfasst.

Es ist bevorzugt, die erfindungsgemäßen Verbindungen oder den Kit, der diese umfasst, in einer Kombinationstherapie, insbesondere zur Behandlung von Tumoren bei Organismen - bevorzugt Mensch oder Tier - zu verwenden. Die Behandlung von Tumoren umfasst die Prophylaxe, Prävention, Diagnose, Verminderung, Therapie, Verlaufskontrolle und/oder Nachbehandlung einer Metastasierung, einer Invasion und/oder einer Angiogenese, wobei die Verlaufskontrolle bevorzugt die Überwachung der Wirksamkeit einer Antitumorbehandlung ist. Besonders bevorzugt ist hierbei, dass die Kombinationstherapie eine Chemotherapie, eine Zytostatikabehandlung und/oder eine Strahlentherapie umfasst. In einer besonders bevorzugten Ausführungsform der Erfindung ist die Kombinationstherapie eine adjuvante biologisch-spezifizierte Therapieform. Ganz besonders bevorzugt ist hierbei, dass diese Therapieform eine Immuntherapie ist. Weiterhin ist besonders bevorzugt, dass die Kombinationstherapie eine Gentherapie und/oder eine Therapie mit einer erfindungsgemäßen Verbindung umfasst. Dem Fachmann sind verschiedene Kombinationstherapien, insbesondere zur Behandlung von Tumoren, bekannt. Es kann zum Beispiel vorgesehen sein, dass innerhalb einer Kombinationstherapie eine Zytostatikabehandlung erfolgt oder beispielsweise eine Bestrahlung eines bestimmten Tumorareals, wobei diese Behandlung mit einer Gentherapie kombiniert wird, wobei die erfindungsgemäßen Verbindungen als Antikrebsmittel eingesetzt werden. Demgemäß kann es ganz besonders bevorzugt sein, dass die erfindungsgemäßen Verbindungen zur Erhöhung der Sensitivität von Tumorzellen gegenüber Zytostatika und/oder Strahlen verwendet werden. Weiterhin ist es bevorzugt, dass die erfindungsgemäßen Verbindungen zur Hemmung der Vitalität, der Proliferationsrate von Zellen und/oder zur Induktion von Apoptose und eines Zellzyklus-Arrests verwendet werden.

In einer bevorzugten Ausführungsform ist die Krebserkrankung oder der Tumor, die/der behandelt oder verhindert wird, ausgewählt aus der Gruppe von Krebserkrankungen oder Tumorerkrankungen des Hals-Nasen-Ohren-Bereichs, der Lunge, des Mediastinums, des Gastrointestinaltraktes, des Urogenitalsystems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindesalter, Lymphomen, Leukämien, paraneoplastischen Syndromen, Metastasen ohne bekannten Primärtumor (CUP-Syndrom), peritonealen Karzinomastosen, Immunsuppressionbezogenen Malignitäten und/oder Tumor-Metastasen.

Insbesondere kann es sich bei den Tumoren um folgende Krebsarten handeln: Adenokarzinom der Brust, der Prostata und des Dickdarms; alle Formen von Lungenkrebs, der von den Bronchien ausgeht; Knochenmarkkrebs, das Melanom, das Hepatom, das Neuroblastom; das Papillom; das Apudom, das Choristom, das Branchiom; das maligne Karzinoid-Syndrom; die Karzinoid-Herzerkrankung; das Karzinom (zum Beispiel Walker-Karzinom, Basalzellen-Karzinom, basosquamöses Karzinom, Brown-Pearce-Karzinom, duktales Karzinom, Ehrlich-Tumor, in situ-Karzinom, Krebs-2-Karzinom, Merkel-Zellen-Karzinom, Schleimkrebs, nicht-kleinzelliges Bronchialkarzinom, Haferzellen-Karzinom, papilläres Karzinom, szirrhöses Karzinom, bronchiolo-alveoläres Karzinom, Bronchiai-Karzinom, Plattenepithelkarzinom und Transitionalzell-Karzinom); histiocytische Funktionsstörung; Leukämie (zum Beispiel in Zusammenhang mit B-Zellen-Leukämie, Gemischt-Zellen-Leukämie, Nullzellen-Leukämie, T-Zellen-Leukämie, chronische T-Zellen-Leukämie, HTLV-II-assoziierte Leukämie, akut lymphozytische Leukämie, chronisch-lymphozythische Leukämie, Mastzell-Leukämie und myeloische Leukämie); maligne Histiocytose, Hodgkin-Krankheit, non-Hodgkin-Lymphom, solitärer Plasmazelltumor; Reticuloendotheliose, Chondroblastom; Chondrom, Chondrosarkom; Fibrom; Fibrosarkom; Riesenzell-Tumore; Histiocytom; Lipom; Liposarkom; Leukosarkom; Mesotheliom; Myxom; Myxosarkom; Osteom; Osteosarkom; Ewing-Sarkom; Synoviom; Adenofribrom; Adenolymphom; Karzinosarkom, Chordom, Craniopharyngiom, Dysgerminom, Hamartom; Mesenchymom; Mesonephrom, Myosarkom, Ameloblastom, Cementom; Odontom; Teratom; Thymom, Chorioblastom; Adenokarzinom, Adenom; Cholangiom; Cholesteatom; Cylindrom; Cystadenocarcinom, Cystadenom; Granulosazelltumor; Gynadroblastom; Hidradenom; Inselzelltumor; Leydig-Zelltumor; Papillom; Sertoli-Zell-Tumor, Thekazelltumor, Leiomyom; Leiomyosarkom; Myoblastom; Myom; Myosarkom; Rhabdomyom; Rhabdomyosarkom; Ependynom; Ganglioneurom, Gliom; Medulloblastom, Meningiom; Neurilemmom; Neuroblastom; Neuroepitheliom, Neurofibrom, Neurom, Paragangliom, nicht-chromaffines Paragangliom, Angiokeratom, angiolymphoide Hyperplasie mit Eosinophilie; sclerosierendes Angiom; Angiomatose; Glomangiom; Hemangioendotheliom; Hemangiom; Hemangiopericytom, Hemangiosarkom; Lymphangiom, Lymphangiomyom, Lymphangiosarkom; Pinealom; Cystosarkom phyllodes; Hemangiosarkom; Lymphangiosarkom; Myxosarkom, Ovarialkarzinom; Sarkom (zum Beispiel Ewing-Sarkom, experimentell, Kaposi-Sarkom und Mastzell-Sarkom); Neoplasmen (zum Beispiel Knochen-Neoplasmen, Brust-Neoplasmen, Neoplasmen des Verdauungssystems, colorektale Neoplasmen, Leber-Neoplasmen, Pankreas-Neoplasmen, Hirnanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des Urogenitaltrakts); Neurofibromatose und zervikale Plattenepitheldysplasie.

In einer weiter bevorzugten Ausführungsform ist die Krebserkrankung oder der Tumor, die/der behandelt oder verhindert wird, ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantations-bezogene Malignitäten, metastasierte Tumoren umfassend Gehirnmetastasen, Lungenmetastasen, Lebernetastasen, Knochenmetastasen, pleurale und perikardiale Metastasen und maligne Aszites.

In einer weiter bevorzugten Ausführungsform ist die Krebserkrankung oder der Tumor, die/der behandelt oder verhindert wird, ausgewählt aus der Gruppe umfassend Krebserkrankungen oder Tumorerkrankungen der Mammakarzinome, der Gastrointestinaltumore, einschließlich Kolonkarzinome, Magenkarzinome, Pankreaskarzinome, Dickdarmkrebs, Dünndarmkrebs, der Ovarialkarzinome, der Zervikalkarzinome, Lungenkrebs, Prostatakrebs, Nierenzellkarzinome und/oder Lebermetastasen.

Die erfindungsgemäßen pharmazeutischen Mittel weisen gegenüber den bekannten Verbindungen oder Mitteln insbesondere gegenüber cis-Platin-Verbindungen, zahlreiche Vorteile auf.

Die erfindungsgemäßen cis-Oxoplatin-Verbindungen können in einem größeren Konzentrationsspektrum eingesetzt werden als cis-Platin-Verbindungen. Auch ist die letale Dosis von cis-Oxoplatin proportional betrachtet viel höher als die von cis-Platin-Verbindungen. Die Wirksamkeit und die Effektivität von cis-Oxoplatin auf bestimmte Tumorarten ist größer als die von cis-Platin.

So konnte beispielsweise gezeigt werden, dass bestimmte Prostatakrebsformen, die gegen cis-Platin-Verbindungen resistent sind, bei der Behandlung von cis-Oxoplatin vorteilhafte Effekte zeigen. Weiterhin ist der nephrotoxische Effekt von cis-Oxoplatin im Vergleich zu cis-Platin viel geringer und die antimetastatische Wirkung von cis-Oxoplatin ist höher als die von cis-Platin. Dies hat seine Ursache unter anderem auch darin, dass cis-Oxoplatin-Verbindungen aufgrund ihrer anderen räumlichen Struktur beispielsweise auf eine andere Art und Weise mit DNA-Molekülen wechselwirken. So basiert beispielsweise die Bindung ausgewählter cis-Platin-Verbindungen an DNA-Molekülen auf einer inneren Substitution von Chlorliganden, währenddessen die Bindung von cis-Oxoplatin mit DNA-Molekülen auf der Ausbildung von Wasserstoffbrückenbindungen basiert. Im Körper eines Patienten, der ein Mensch oder ein Tier sein kann, zeigen cis-Oxoplatin-Verbindungen weitgehend inertes Verhalten. Aus diesem Grunde können sie in höheren Konzentrationen ohne toxische Nebeneffekte eingesetzt werden als cis-Platin-Verbindungen. Eine weitere Besonderheit der erfindungsgemäßen pharmazeutischen Mittel, die cis-Oxoplatin umfassen, ist, dass sie gegen nahezu alle Arten von Tumoren eine Antitumorwirksamkeit zeigen. Die erfindungsgemäßen pharmazeutischen Mittel haben insbesondere in der Leber und in der Niere eine geringere Halbwertszeit als pharmazeutische Mittel, die auf cis-Platin-Verbindungen basieren. Besonders vorteilhaft ist es, dass die erfindungsgemäßen Mittel eine sehr gute Wirkung zeigen, wenn sie oral gegeben werden, da sie sehr schnell in eine systemische Zirkulation innerhalb des Körpers übergehen, was zu einer deutlichen Erhöhung ihrer Antitumoraktivität, die sich beispielsweise in der Reduktion der Tumorgröße zeigt, führt. Weiterhin können die erfindungsgemäßen Salben, Cremes und Gele mit sehr gutem Erfolg in der topischen Chemotherapie eingesetzt werden. Bei dieser topischen Chemotherapie wird beispielsweise die erfindungsgemäße Salbe, die Creme bzw. das Gel oder aber auch ein Puder direkt auf die Hautoberfläche aufgetragen. Dies kann auch dann sinnvoll sein, wenn sich noch kein Tumor ausgebildet hat, sich aber bereits bestimmte Vorformen, wie zum Beispiel Akrokeratosen manifestiert haben. Diese Anwendung ist deshalb besonders vorteilhaft, da nicht der gesamte Organismus von eventuell auftretenden Nebenwirkungen betroffen ist. Vorteilhafterweise ist die Haut in der Lage, die erfindungsgemäßen pharmazeutischen Mittel in Form eines Depots aufzunehmen, das heißt, die Halbwertszeit des Wirkstoffes der erfindungsgemäßen Mittel in der Haut beträgt ungefähr 12 Tage. Die erfindungsgemäßen pharmazeutischen Mittel können weiterhin bevorzugt in der intraperitonealen Chemotherapie eingesetzt werden. Durch diese Therapie werden insbesondere abdominale Tumorerkrankungen behandelt. Derartige Chemotherapien können beispielsweise mit einer hyperthermischen intraperitonealen Chemotherapie kombiniert werden. Die hierbei auftretenden hyperthermischen Effekte lassen die erfindungsgemäßen pharmazeutischen Mittel sensitiver auf die Tumoren wirken. Die intraperitoneale Chemotherapie kann insbesondere bei dem Ovarialkarzinom angewendet werden.

Im Folgenden soll die Erfindung anhand von Beispielen näher erläutert werden.

### 1. Anwendung von cis-Diammoniumdichlorodihydroxoplatin (IV) und seinen Salzen

Wachstumsinhibitionsversuche an verschiedenen humanen Zelllinien zeigen die unterschiedlichen Aktivitäten von cis-Platin, cis-Oxoplatin und Oxaliplatin. Die nachfolgend aufgeführten Ergebnisse zeigen, dass cis-Oxoplatin eine ähnliche Aktivität wie Oxaliplatin aufweist, aber eine größere Aktivität als Carboplatin besitzt. Die folgende Tabelle gibt die Ergebnisse wieder, die mit cis-Platin, Oxoplatin, Carboplatin und Oxaliplatin generiert wurden (die angegebenen Werte sind IC50-Werte in µg/ml, das heißt, die Konzentration, bei der 50 % der Zellen überleben, nd = nicht bestimmt, res = resistent = nicht-sensitiv oder ein IC50-Wert kann bei einer Konzentration bis zu 40 µg/ml nicht bestimmt werden; IC = Inhibitions-Konzentration).

**Tabelle 1**

| **Zelllinie** | **cis-Platin** | **cis-Oxoplatin** | **Carbo-platin** | **Oxali-platin** |
|---|---|---|---|---|
| HOS Osteosarcoma | nd | 2.5 | 5 | nd |
| SaOS Osteosarcoma | nd | 5 | 5 | nd |
| PC3 Prostate | res | 7.5 | 10 | nd |
| M607 Melanoma | 0.3 | 5 | 10 | 10 |
| M518 Melanoma | 40 | res | res | res |
| Me128 Melanoma | 0.3 | 2.5 | 10 | 10 |
| JVSO Melanoma | 40 | 10 | res | res |
| Pancl Pancreatic cancer | 1 | 40 | 20 | 5 |
| BxPC3 Pancreatic cancer | 0.6 | 2.5 | 10 | 10 |
| MiaPaCa2 Pancreatic cancer | 1.5 | 5 | 5 | 5 |
| HCT8 Colon carcinoma | 5 | 40 | res | res |
| HT29 Colon carcinoma | 0.3 | 20 | 20 | 20 |
| HCT-15 Colon carcinoma | 0.3 | 20 | res | 10 |
| A498 Renal cells | 1 | 20 | res | 10 |
| C320DM Colon carcinoma | 0.3 | 2.5 | 10 | 0.15 |
| Colo205 Colon carcinoma | 10 | res | res | 1 |
| CC1227 Colon carcinoma | 0.3 | 10 | res | 0.2 |
| MCF-7 Brust cancer | 2.5 | 5.5 | res | res |
| T47D Brust cancer | 0.3 | 2.5 | nd | 0.1 |

**Tabelle 2**

| **Zelllinie** | **IC50 µg/ml** | |
|---|---|---|
| | **cis-Oxoplatin** | **Cis-Oxoplatin-Natriumsalz** |
| T47D Brust cancer | 3 | 18 |
| SK-OV3 Ovarian cancer | 15 | 22 |
| U 373 MG Astrocytoma | 15 | 18 |
| BxPC3 Pancreatic carcinoma | 13 | 12 |

Die ermittelten Ergebnisse zeigen, dass chemisch sehr ähnliche Platinverbindungen, wie zum Beispiel cis-Platin und cis-Oxoplatin, unterschiedliche Wirkungen auf verschiedene Humankrebszellen haben und dass die Salze der Platinverbindungen ein anderes Verhalten auf Tumoren zeigen als die Basenverbindungen, aus denen die Salze generiert wurden. Ganz allgemein und über die konkreten Versuche hinausgehend zeigt sich, dass die DNA-Bindungsfähigkeiten von cis-Oxoplatin-Salzen, insbesondere von cis-Oxoplatin-Natriumsalz, unerwartet gegenüber denen von cis-Oxoplatin sind. Dies kann beispielsweise seine Ursache in der unterschiedlichen Struktur der DNA-Addukte haben, die einerseits mit der Base und zum anderen mit dem Salz gebildet werden. Weiterhin kann vermutet werden, dass die cis-Oxoplatin-Salze einen anderen Prozess der Biotransformation durchlaufen als die entsprechenden Basen. Diese unerwarteten Unterschiede haben eine große Bedeutung beim Einsatz in der Tumortherapie von Basen und Salzen. Weitere wichtige Punkte des unterschiedlichen Verhaltens von Basen und Salzen sind zum Beispiel: die Aufnahme, die Ausbreitung und die Verteilung im Gewebe sowie in einzelnen Organen. Die intrazelluläre Aufnahme und die Toxizität der cis-Oxoplatin-Natriumsalze ist eine andere als die der entsprechenden Base; die Absorption und die Auflösung sowie die Pharmakogenetik der cis-Oxoplatin-Salze ist nicht mit denen der Base vergleichbar. Die Art der Wechselwirkung mit der DNA wie auch die Effizienz und die Wirksamkeit sowie auch die therapeutische Potenz der cis-Oxoplatin-Salze ist eine andere als die von cis-Oxoplatin. Dies kann unter anderem an der chemischen Struktur von cis-Oxoplatin-Calziumsalzen als ein Beispiel von Salzen aus zweiwertigen Kationen gezeigt werden: 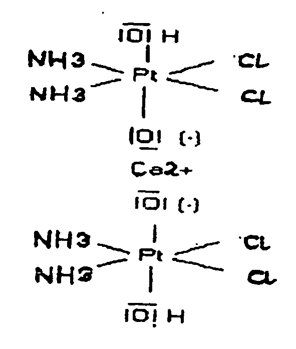 An dieser Struktur zeigt sich, dass Salze wie zum Beispiel Calziumsalze eine vollkommen andere Struktur als die entsprechende Base haben. Diese unterschiedlichen stereochemischen Eigenschaften bewirken ein anderes Verhalten bei der Wechselwirkung mit der DNA in Zellen, insbesondere Krebszellen. Durch die unterschiedliche Struktur der Salze kann eine geringere Dosis ausreichend sein, um einen therapeutischen Effekt zu erzielen. Weiterhin kann die Biotransformation dazu führen, dass die Platin(IV)-Komplexe im Körper zu Platin(II)-Komplexen umgebildet werden, wobei die Platin(IV)- und die Platin(II)-Komplexe auf unterschiedliche Tumoren unterschiedlich wirken (siehe Tabelle I).

### 2. Zytotoxische Aktivität von trans-Oxoplatin(TRAXO)

Trans-Oxoplatin wurde mit einer Anfangskonzentration von 40 g/ml und 2-fachen Schritten gegen Panel f Zelllinien getestet. Da die IC50-Werte in den meisten Fällen nicht erreicht wurden, ist das Überleben der Zellen bei der höchsten Konzentration angegeben.

**Tabelle 3**

| **Zelllinie** | | **% Überleben bei 40 µg/ml TRAXO (außer wenn 20 µm/ml angezeigt sind)** |
|---|---|---|
| U-87-MG | Astrocytoma | 100 |
| ASTRO | Astrocytoma | 82(20)/71 |
| SW620 | Colon carcinoma | 43/51/97 |
| MDA-MB-231 | Brust cancer | 70/103/106 |
| G-292 | Osteosarcoma | 8.6/68 |
| PANC1 | Pancreatic cancer | 100 |
| CRO1A | Carcinoid | 87/104/70 |
| CR02B | Carcinoid | 24/57 |
| MIAPaCa2 | Pancreatic Cancer | 92/83 |
| Fib 3 | Fibroblasts | 91 |
| K562 | Leukaemia | 97 |
| WI-38 | Embry, lung fibroblasts | 21 |
| COLO 205 | Colon carcinoma | 109 |
| HCT-15 | Colon carcinoma | 100 |
| T-47D | Brust cancer | 101 |
| HL-60 | Leukaemia | 0.5 |
| HOS | Osteosarcoma | 4.3 |
| ACHN | Renal carcinoma | 48 |
| BxPC3 | Pancreatic carcinoma | 106 |

Wie durch die Versuche an 19 Zelllinien gezeigt wird, hat TRAXO eine erhebliche Aktivität gegen eine Colon Karzinomzelllinie (SW 620), gegen 2 Osteosarcom-Zelllinien (G-292, HOS), gegen eine Nierenkarzinom-Zelllinie (ACHN), eine Leukämiezelllinie (HL-60) und eine embryonale Lungenfibroblastenzelllinie (WI-38). Zelllinien, die gegen cis-Oxoplatin sensitiv sind, wie beispielsweise T-47D und BxPC3, sind nicht sensitiv gegenüber TRAXO. Die Salze der trans-Oxoplatinverbindungen können ein unterschiedliches therapeutisches Potential und eine andere Wirksamkeit gegen einzelne humane Krebszellen und Zelllinien sowie Tumoren haben.

## Patentansprüche

1. Verfahren zur Herstellung eines pharmazeutischen Mittels umfassend cis-Diammoniumdichlorotransdihydroxoplatin (IV) oder dessen Salze
**dadurch gekennzeichnet, dass**
das pharmazeutische Mittel eine Tablette, eine Kapsel, ein Dragee, ein Zäpfchen, eine Salbe, eine Creme, eine Injektions- und/oder eine Infusionslösung ist, wobei die Kapsel Oxoplatin : Siliciumdioxid : Mannitol oder Magnesiumstearat im Verhältnis von 0,1 bis 10 : 0,1 bis 10 : 0,1 bis 10 umfasst,
die Tablette cis-Oxoplatin : Lactose : Maisstärke : Poly (o-Carboxymethyl) stärke-Natriumsalz : Calciumhydrogenphosphat 2H₂O : Cellulosepulver : Magnesiumstearat im Verhältnis von 10 bis 500 : 20 bis 150 : 1 bis 10 : 1 bis 10 : 1 bis 10 : 1 bis 10 : 0,1 bis 7 umfasst,
oder die Tablette alternativ cis-Oxoplatin : Siliciumdioxid : Magnesiumstearat im Verhältnis von 0,1 bis 10 : 0,1 bis 10 : 0,1 bis 10 umfasst,
die Creme cis-Oxoplatin : Benzylalkohol : Cestylstearylalkohol : Macrogolstearat 1000 : Isopropylpalmitat : Glycerol : Sorbitol-Lösung 70 % : Wasser im Verhältnis von 0,2 bis 8 : 0,1 bis 7 : 1 bis 10 : 0,1 bis 7 : 0,1 bis 7 : 0,2 bis 8 : 0,2 bis 8 : 20 bis 60 umfasst,
die Salbe cis-Oxoplatin : Propylenglykol : Macrogolstearat 1000 : Cestylstearylalkohol : Vaseline im Verhältnis von 2 bis 20 : 5 bis 40 : 0,1 bis 7 : 1 bis 10 : 25. bis 400 umfasst,
das Gel cis-Oxoplatin : Hydroxyethylcellulose : Chlorocresol : Natriumhydroxid : Natriumhydrogenphosphatdihydrat : Wasser im Verhältnis von 2 bis 20 : 100 bis 600 : 5 bis 40 : 0,1 bis 7 : 20 bis 60 : 3000 bis 50000 umfasst,
das Zäpfchen *cis*-Oxoplatin : Siliciumdioxid : Hartfett im Verhältnis von 0,1 bis 10 : 0,1 bis 10 : 30 bis 300 umfasst,
oder das Zäpfchen alternativ cis-Oxoplatin : Lactose : Maisstärke : Adipinsäure : Natriumhydrogencarbonat : Stearinsäure : Magnesiumstearat : hochdispersem Siliciumdioxid : Polysorbat 80 im Verhältnis von 10 bis 100 : 700 bis 4000 : 200 bis 600 : 10 bis 1000 : 10 bis 1000 : 1 bis 100 : 1 bis 100 : 1 bis 15 :" 0,1 bis 10 umfasst,
oder das Zäpfchen alternativ cis-Oxoplatin : Lactose 1H₂O : Maisstärke : Adipinsäure : Natriumhydrogencarbonat : Stearinsäure : Magnesiumstearat : Siliciumdioxid : Polysorbat 80 im Verhältnis von 10 bis 100 : 1000 bis 5000 : 300 bis 1000 : 10 bis 1000 : 10 bis 1000 : 1 bis 100 : 1 bis 100 : 1 bis 15 : 0,1 bis 7 umfasst,
oder das Zäpfchen alternativ cis-Oxoplatin : Lactose IH2O : Maisstärke : Adipinsäure : Natriumhydrogencarbonat : Stearinsäure : Magnesiumstearat : Siliciumdioxid : Polysorbat 80 im Verhältnis von 10 bis 1000 : 1500 bis 5000 : 300 bis 1000 : 10 bis 1000 : 10 bis 1000 : 1 bis 100 : 1 bis 100 : 1 bis 15 : 0,1 bis 7 umfasst,
die Injektions- oder Infusionslösung cis-Oxoplatin : Benzylalkohol : Polysorbat 80 : Sorbitollösung 70 % : Wasser im Verhältnis von' 0,2 bis 8 : 1 bis 10 : 0,1 bis 7 : 100 bis 800 : 100 bis 400 umfasst,
oder die Injektions- oder Infusionslösung alternativ cis-Oxoplatin : Mannitol : Wasser im Verhältnis von 0,1 bis 7 : 5 bis 40 : 1 bis 10 umfasst,
wobei zunächst der Grundstoff des pharmazeutischen Mittels ohne cis-Diammonium-dichlorotransdihydroxoplatin (IV) bereitgestellt wird und vor der pharmazeutischen Anwendung die angegebene Menge cis-Diammonium-dichlorotransdihydroxoplatin (IV) in den bereitgestellten Grundstoff eingebracht wird.

2. Pharmazeutisches Mittel, herstellbar mit dem Verfahren gemäß Anspruch 1.

3. Pharmazeutisches Mittel nach Anspruch 2, **dadurch gekennzeichnet, dass** es ein chemotherapeutisches Mittel ist.

4. Pharmazeutisches Mittel nach einem der vorhergehenden Ansprüchen 2 bis 3,
**dadurch gekennzeichnet, dass**
es zusätzlich Siliciumdioxid und Mannitol oder Siliciumdioxid und Magnesiumstearat und/oder pharmazeutisch akzeptierbare Vehikel, insbesondere Siosomen, Liposomen und/oder Nanokapseln umfasst.

5. Pharmazeutisches Mittel nach einem der vorhergehenden Ansprüchen 2 bis 4,
**dadurch gekennzeichnet, dass**
die Kapsel 50 mg Siliciumdioxid, 50 mg Mannitol oder 50 mg Magnesiumstearat und 50 mg Oxoplatin umfasst, oder alternativ, 50 mg cis-Oxoplatin, 39,5 mg Lactose oder 39 mg, 2,5 mg oder 2 mg Maisstärke, 2,5 mg Poly(o-Carboxymethyl)stärke-Natriumsalz, 2,5 mg Calciumhydrogenphosphat 2H₂O, 2,5 mg Cellulosepulver und 0,5 mg Magnesiumstearat umfasst, oder alternativ, 50 mg cis-Oxoplatin, 50 mg Siliciumdioxid und 50 mg Magnesiumstearat umfasst.

6. Pharmazeutisches Mittel nach einem der vorhergehenden Ansprüchen 2 bis 5,
**dadurch gekennzeichnet, dass**
die Creme 50 mg cis-Oxoplatin, 20 mg Benzylalkohoi, 100 mg Cestylstearylalkohol, 25 mg Macrogolstearat 1000, 20 mg Isopropylpalmitat, 40 mg Glycerol, 50 mg Sorbitol und 205 mg Wasser umfasst.

7. Pharmazeutisches Mittel nach einem der vorhergehenden Ansprüchen 2 bis 6,
**dadurch gekennzeichnet, dass**
die Salbe 50 mg cis-Oxoplatin, 120 mg Propylenglykol, 5,5 mg Macrogolstearat 1000, 22 mg Cestylstearylalkohol und 851,5 mg Vaseline umfasst.

8. Pharmazeutisches Mittel nach einem der vorhergehenden Ansprüchen 2 bis 7,
**dadurch gekennzeichnet, dass**
das Gel 0,05 g cis-Oxoplatin, 1,8 g Hydroxyethylcellulose, 0,1 g Chloroaerosol, 0,005 g Natriumhydroxid, 0,17 g Natriumhydrogenphosphatdihydrat und 97,875 g Wasser umfasst.

9. Pharmazeutisches Mittel nach einem der vorhergehenden Ansprüchen 2 bis 8,
**dadurch gekennzeichnet, dass** das Zäpfchen 0,02 g cis-Oxoplatin, 0,02 g Siliciumdioxid und 1,85 g Hartfett umfasst, alternativ, dass das Zäpfchen 20 mg cis-Oxoplatin, 1055 mg Lactose, 170 mg Maisstärke, 63,60 mg Adipinsäure, 50 mg Natriumhydrogencarbonat, 5 mg Stearinsäure, 4,5 mg Magnesiumstearat, 3 mg hochdisperses Siliciumdioxid und 0,5 mg Polysorbat 8 0 umfasst, alternativ, dass das Zäpchen 20 mg cis-Oxoplatin, 1350 mg Lactose 1H₂O, 170 mg Maisstärke, 65 mg Adipinsäure, 50 mg Natriumhydrogencarbonat, 5 mg Stearinsäure, 4,5 mg Magnesiumstearat, 3 mg hochdisperses Siliciumdioxid und 0,5 mg Polysorbat 80 umfasst, oder alternativ, dass das Zäpfchen 50 mg cis-Oxoplatin, 1450 mg Lactose 1H₂O, 170 mg Maisstärke, 65 mg Adipinsäure, 50 mg Natriumhydrogencarbonat, 5 mg Stearinsäure, 4,5 mg Magnesiumstearat, 3 mg hochdisperses Siliciumdioxid und 0,5 mg Polysorbat 80 umfasst.

10. Pharmazeutisches Mittel nach einem der vorhergehenden Ansprüchen 2 bis 9,
**dadurch gekennzeichnet, dass**
die Präparation einer 5 mg/ml Injektions- oder Infusionslösung 5 mg cis-Oxoplatinᵣ 9 mg Benzylalkohol, 2 mg Polysorbat 80, 650 mg Sorbitollösung 70% und 500 mg Wasser oder 5 mg cis-Oxoplatin, 100 mg Mannitol und 200 ml Wasser umfasst.

11. Pharmazeutisches Mittel nach einem der vorhergehenden Ansprüchen 2 bis 10,
**dadurch gekennzeichnet, dass**
die Tablette 50 mg cis-Oxoplatin, 39,5 mg Lactose, 2,5 mg Maisstärke, 2,5 mg Poly(o-Carboxymethyl)stärke-Natriumsalz, 2,5 mg Calciumhydrogenphosphat 2H₂O, 2,5 mg Cellulosepulver und 0,5 mg Magnesiumstearat umfasst oder 50 mg cis-Oxoplatin, 39 mg Lactose, 2 mg Maisstärke, 2,5 mg Poly(o-Carboxymethyl)stärke-Natriumsalz, 2,5 mg Calciumhydrogenphosphat 2H₂O, 2,5 mg Cellulosepulver und 0,5 mg Magnesiumstearat umfasst oder 50 mg cis-Oxoplatin, 50 mg Siliciumdioxid und 50 mg Magnesiumstearat umfasst.

12. Verwendung der pharmazeutischen Mittel nach einem der Ansprüche 2 bis 11 zur Herstellung eines Arzneimittels zur Behandlung von Tumoren.

## Claims

1. A method for the production of a pharmaceutical agent comprising *cis*-diammoniumdichloro-*trans*-dihydroxoplatinum(IV) or salts thereof,
**characterized in that**
the pharmaceutical agent is a tablet, a capsule, a coated tablet, a suppository, an ointment, a cream, a solution for infusion and/or injection,
said capsule comprising oxoplatin : silicon dioxide : mannitol or magnesium stearate at a ratio of 0.1 to 10 : 0.1 to 10 : 0.1 to 10;
said tablet comprising *cis*-oxoplatin : lactose : corn starch : poly(O-carboxymethyl)starch sodium salt : calcium hydrogen phosphate × 2H₂O : cellulose powder : magnesium stearate at a ratio of 10 to 500 : 20 to 150 : 1 to 10 : 1 to 10 : 1 to 10 : 1 to 10 : 0.1 to 7; or
said tablet alternatively comprising *cis*-oxoplatin : silicon dioxide : magnesium stearate at a ratio of 0.1 to 10 : 0.1 to 10 : 0.1 to 10;
said cream comprising *cis*-oxoplatin : benzyl alcohol : cetyl stearyl alcohol : Macrogol stearate 1000 : isopropyl palmitate : glycerol : 70% sorbitol solution : water at a ratio of 0.2 to 8 : 0.1 to 7 : 1 to 10 : 0.1 to 7 : 0.1 to 7 : 0.2 to 8 : 0.2 to 8 : 20 to 60;
said ointment comprising *cis*-oxoplatin : propylene glycol : Macrogol stearate 1000 : cetyl stearyl alcohol : vaseline at a ratio of 2 to 20 : 5 to 40 : 0.1 to 7 : 1 to 10 : 25 to 400;
said gel comprising *cis*-oxoplatin : hydroxyethylcellulose : chlorocresol : sodium hydroxide : sodium hydrogen phosphate dihydrate : water at a ratio of 2 to 20 : 100 to 600 : 5 to 40 : 0.1 to 7 : 20 to 60 : 3,000 to 50,000;
said suppository comprising *cis*-oxoplatin : silicon dioxide : hardened fat at a ratio of 0.1 to 10 : 0.1 to 10 : 30 to 300; or
said suppository alternatively comprising *cis*-oxoplatin : lactose : corn starch : adipic acid : sodium hydrogen carbonate : stearic acid : magnesium stearate : highly dispersed silicon dioxide : Polysorbate 80 at a ratio of 10 to 100 : 700 to 4,000 : 200 to 600 : 10 to 1,000 : 10 to 1,000 : 1 to 100 : 1 to 100 : 1 to 15 : 0.1 to 10; or
said suppository alternatively comprising *cis*-oxoplatin : lactose × 1H₂O : corn starch : adipic acid : sodium hydrogen carbonate : stearic acid : magnesium stearate : silicon dioxide : Polysorbate 80 at a ratio of 10 to 100 : 1,000 to 5,000 : 300 to 1,000 : 10 to 1,000 : 10 to 1,000 : 1 to 100 : 1 to 100 : 1 to 15 : 0.1 to 7; or said suppository alternatively comprising *cis*-oxoplatin : lactose × 1H₂O : corn starch : adipic acid : sodium hydrogen carbonate : stearic acid : magnesium stearate : silicon dioxide : Polysorbate 80 at a ratio of 10 to 1,000 : 1,500 to 5,000 : 300 to 1,000 : 10 to 1,000 : 10 to 1,000 : 1 to 100 : 1 to 100 : 1 to 15 : 0.1 to 7;
said solution for injection or infusion comprising *cis-*oxoplatin : benzyl alcohol : Polysorbate 80 : 70% sorbitol solution : water at a ratio of 0.2 to 8 : 1 to 10 : 0.1 to 7 : 100 to 800 : 100 to 400; or
said solution for injection or infusion alternatively comprising *cis*-oxoplatin : mannitol : water at a ratio of 0.1 to 7 : 5 to 40 : 1 to 10;
in which method the base material of the pharmaceutical agent is initially provided with no *cis*-diammoniumdichloro-trans-dihydroxoplatinum(IV) and the specified amount of cis-diammoniumdichloro-trans-dihydroxoplatinum(IV) is incorporated in the provided base material prior to pharmaceutical application.

2. A pharmaceutical agent, which agent can be produced by means of the method according to claim 1.

3. The pharmaceutical agent according to claim 2,
**characterized in that**
said agent is a chemotherapeutic agent.

4. The pharmaceutical agent according to any of the preceding claims 2 to 3,
**characterized in that**
the capsule additionally comprises silicon dioxide and mannitol or silicon dioxide and magnesium stearate and/or pharmaceutically acceptable vehicles, especially siosomes, liposomes and/or nanocapsules.

5. The pharmaceutical agent according to any of the preceding claims 2 to 4,
**characterized in that**
the capsule comprises 50 mg of silicon dioxide, 50 mg of mannitol or 50 mg of magnesium stearate and 50 mg of oxoplatin, or, alternatively, 50 mg of *cis*-oxoplatin, 39.5 mg of lactose or 39 mg, 2.5 mg or 2 mg of corn starch, 2.5 mg of poly(O-carboxymethyl)starch sodium salt, 2.5 mg of calcium hydrogen phosphate × 2H₂O, 2.5 mg of cellulose powder, and 0.5 mg of magnesium stearate, or, alternatively, 50 mg *cis*-oxoplatin, 50 mg of silicon dioxide and 50 mg of magnesium stearate.

6. The pharmaceutical agent according to any of the preceding claims 2 to 5,
**characterized in that**
the cream comprises 50 mg of *cis*-oxoplatin, 20 mg of benzyl alcohol, 100 mg of cetyl stearyl alcohol, 25 mg of Macrogol stearate 1000, 20 mg of isopropyl palmitate, 40 mg of glycerol, 50 mg of sorbitol and 205 mg of water.

7. The pharmaceutical agent according to any of the preceding claims 2 to 6,
**characterized in that**
the ointment comprises 50 mg of *cis*-oxoplatin, 120 mg of propylene glycol, 5.5 mg of Macrogol stearate 1000, 22 mg of cetyl stearyl alcohol, and 851.5 mg of vaseline.

8. The pharmaceutical agent according to any of the preceding claims 2 to 7,
**characterized in that**
the gel comprises 0.05 g of *cis*-oxoplatin, 1.8 g of hydroxyethylcellulose, 0.1 g of chloroaerosol, 0.005 g of sodium hydroxide, 0.17 g of sodium hydrogen phosphate dihydrate, and 97.875 g of water.

9. The pharmaceutical agent according to any of the preceding claims 2 to 8,
**characterized in that**
the suppository comprises 0.02 g of *cis*-oxoplatin, 0.02 g of silicon dioxide and 1.85 g of hardened fat, and alternatively, that the suppository comprises 20 mg of *cis*-oxoplatin, 1055 mg of lactose, 170 mg of corn starch, 63.60 mg of adipic acid, 50 mg of sodium hydrogen carbonate, 5 mg of stearic acid, 4.5 mg of magnesium stearate, 3 mg of highly dispersed silicon dioxide and 0.5 mg of Polysorbate 80, and alternatively, that the suppository comprises 20 mg of *cis*-oxoplatin, 1350 mg of lactose × 1H₂O, 170 mg of corn starch, 65 mg of adipic acid, 50 mg of sodium hydrogen carbonate, 5 mg of stearic acid, 4.5 mg of magnesium stearate, 3 mg of highly dispersed silicon dioxide and 0.5 mg of Polysorbate 80, or, alternatively, that the suppository comprises 50 mg of *cis*-oxoplatin, 1450 mg of lactose × 1H₂O, 170 mg of corn starch, 65 mg of adipic acid, 50 mg of sodium hydrogen carbonate, 5 mg of stearic acid, 4.5 mg of magnesium stearate, 3 mg of highly dispersed silicon dioxide and 0.5 mg of Polysorbate 80.

10. The pharmaceutical agent according to any of the preceding claims 2 to 9,
**characterized in that**
the preparation of a 5 mg/ml injection or infusion solution comprises 5 mg of *cis*-oxoplatin, 9 mg of benzyl alcohol, 2 mg of Polysorbate 80, 650 mg of 70% sorbitol solution and 500 mg of water.

11. The pharmaceutical agent according to any of the preceding claims 2 to 10,
**characterized in that**
the tablet comprises 50 mg of *cis*-oxoplatin, 39.5 mg of lactose, 2.5 mg of corn starch, 2.5 mg of poly(O-carboxymethyl)starch sodium salt, 2.5 mg of calcium hydrogen phosphate 2H₂O, 2.5 mg of cellulose powder and 0.5 mg of magnesium stearate, or, alternatively, 50 mg of *cis*-oxoplatin, 39 mg of lactose, 2 mg of corn starch, 2.5 mg poly(O-carboxymethyl)starch sodium salt, 2.5 mg cellulose powder and 0,5 mg of magnesium stearate, or, alternatively, 50 mg of *cis*-oxoplatin, 50 mg of silicon dioxide and 50 mg of magnesium stearate.

12. Use of the pharmaceutical agent according to any of claims 2 to 11 in the production of a drug for the prophylaxis or treatment of tumors.

## Revendications

1. Procédé de préparation d'un agent pharmaceutique contenant du *cis-*diammoniumdichloro-*trans*-dihydroxoplatine(IV) ou ses sels,
**caractérisé en ce que**
l'agent pharmaceutique est un comprimé, une capsule, une dragée, un suppositoire, une pommade, une crème, une solution d'injection et/ou de perfusion, moyennant quoi
la capsule contient de l'oxoplatine : du dioxyde de silicium : du mannitol ou du stéarate de magnésium, dans une proportion respective de 0,1 à 10 : 0,1 à 10 : 0,1 à 10,
le comprimé contient du cis-oxoplatine : du lactose : de l'amidon de maïs : du poly(O-carboxyméthyl)amidon-sel de sodium : de l'hydrogénophosphate de
calcium · 2 H₂O : de la poudre de cellulose : du stéarate de magnésium dans une proportion respective de 10 à 500 : 20 à 150 : 1 à 10 : 1 à 10 : 1 à 10 : 1 à 10:0,1 à7,
ou le comprimé contient d'une autre façon du cis-oxoplatine : du dioxyde de silicium : du stéarate de magnésium dans une proportion respective de 0,1 à 10 : 0,1 à 10 : 0,1 à 10,
la crème contient du cis-oxoplatine : de l'alcool benzylique : de l'alcool cétylstéarylique : du macrogolstéarate 1000 : de l'isopropylpalmitate : du glycérol : de la solution de sorbitol à 70 % : de l'eau dans une proportion respective de 0,2 à 8 : 0,1 à 7 : 1 à 10 : 0,1 à 7 : 0,1 à 7 : 0,2 à 8 : 20 à 60,
la pommade contient du cis-oxoplatine : du propylène glycol : du macrogolstéarate 1000 : de l'alcool cétylstéarylique : de la vaseline dans une proportion respective de 2 à 20 : 5 à 40 : 0,1 à 7 : 1 à 10 : 25 à 400,
le gel contient du cis-oxoplatine : de l'hydroxyéthylcellulose : du chlorocresol : de l'hydroxyde de sodium : de l'hydrogénophosphate de sodium dihydraté : de l'eau dans une proportion respective de 2 à 20 : 100 à 600 : 5 à 40 : 0,1 à 7 : 20 à 60 : 3000 à 50000,
le suppositoire contient du cis-oxoplatine : du dioxyde de silicium : de la graisse solide dans une proportion respective de 0,1 à 10 : 0,1 à 10 : 30 à 300,
ou le suppositoire contient de façon alternative du cis-oxoplatine : du lactose : de l'amidon de maïs : de l'acide adipique : de l'hydrogénocarbonate de sodium : de l'acide stéarique : du stéarate de magnésium : du dioxyde de silicium fortement dispersé : du polysorbate 80 dans une proportion respective de 10 à 100 : 700 à 4000 : 200 à 600 : 10 à 1000 : 10 à 1000 : 1 à 100 : 1 à 100 : 1 à 15 : 0,1 à 10,
ou le suppositoire contient de façon alternative du cis-oxoplatine : du lactose. 1 H₂O : de l'amidon de maïs : de l'acide adipique : de l'hydrogénocarbonate de sodium : de l'acide stéarique : du stéarate de magnésium : du dioxyde de silicium : du polysorbate 80 dans une proportion respective de 10 à 100 : 1000 à 5000 : 300 à 1000 : 10 à 1000 : 10 à 1000 : 1 à 100 : 1 à 100 : 1 à 15 : 0,1 à 7,
ou le suppositoire contient de façon alternative du cis-oxoplatine : du lactose · 1H₂O : de l'amidon de maïs : de l'acide adipique : de l'hydrogénocarbonate de sodium : de l'acide stéarique : du stéarate de magnésium : du dioxyde de silicium : du polysorbate 80 dans une proportion respective de 10 à 1000 : 1500 à 5000 : 300 à 1000 : 10 à 1000 : 10 à 1000 : 1 à 100 : 1 à 100 : 1 à 15 : 0,1 à 7,
la solution d'injection ou de perfusion contient du cis-oxoplatine : de l'alcool benzylique : du polysorbate 80 : de la solution de sorbitol à 70 % : de l'eau dans une proportion respective de 0,2 à 8 : 1 à 10 : 0,1 à 7 : 100 à 800 : 100 à 400,
ou la solution d'injection ou de perfusion contient de façon alternative du cis-oxoplatine : du mannitol : de l'eau dans une proportion respective de 0,1 à 7 : 5 à 40 : 1 à 10,
moyennant quoi on prépare d'abord la préparation de base de l'agent pharmaceutique à l'exception du *cis*-diammoniumdichloro-*trans-*dihydroxoplatine(IV), et on introduit avant l'application pharmaceutique la quantité indiquée de *cis*-diammoniumdichloro-*trans*-dihydroxoplatine(IV) dans la matière de base.

2. Agent pharmaceutique pouvant être préparé avec le procédé selon la revendication 1.

3. Agent pharmaceutique selon la revendication 2,
**caractérisé en ce que**
c'est un agent chimiothérapeutique.

4. Agent pharmaceutique selon l'une des revendications précédentes 2 à 3,
**caractérisé en ce que**
la capsule contient en plus du dioxyde de silicium et du mannitol ou du dioxyde de silicium et du stéarate de magnésium et/ou un véhicule compatible sur un plan pharmaceutique, notamment des siosomes, des liposomes et/ou des nanocapsules.

5. Agent pharmaceutique selon l'une des revendications précédentes 2 à 4,
**caractérisé en ce que**
la capsule contient 50 mg de dioxyde de silicium, 50 mg de mannitol ou 50 mg de stéarate de magnésium et 50 mg d'oxoplatine, ou contient de façon alternative 50 mg de cis-oxoplatine, 39,5 mg de lactose ou 39 mg, 2,5 mg ou 2 mg d'amidon de maïs, 2,5 mg de poly(O-carboxyméthyl)amidon- sel de sodium, 2,5 mg d'hydrogénophosphate de calcium · 2H₂O, 2,5 mg de poudre de cellulose et 0,5 mg de stéarate de magnésium, ou contient de façon alternative 50 mg de cis-oxoplatine, 50 mg de dioxyde de silicium et 50 mg de stéarate de magnésium.

6. Agent pharmaceutique selon l'une des revendications précédentes 2 à 5,
**caractérisé en ce que**
la crème contient 50 mg de cis-oxoplatine, 20 mg d'alcool benzylique, 100 mg d'alcool cétylstéarylique, 25 g de stéarate de macrogol 1000, 20 mg d'isopropylpalmitate, 40 mg de glycérol, 50 mg de sorbitol et 205 mg d'eau.

7. Agent pharmaceutique selon l'une des revendications précédentes 2 à 6,
**caractérisé en ce que**
la pommade contient 50 mg de cis-oxoplatine, 120 mg de propylène glycol, 5,5 g de stéarate de macrogol 1000, 22 mg d'alcool cétylstéarylique et 851,5 mg de vaseline.

8. Agent pharmaceutique selon l'une des revendications précédentes 2 à 7,
**caractérisé en ce que**
le gel contient 0,05 g mg de cis-oxoplatine, 1,8 g d'hydroxyéthylcellulose, 0,1 g de chloroaérosol, 0,005 g d'hydroxyde de sodium, 0,17 g d'hydrogénophosphate de sodium dihydraté et 97,875 g d'eau.

9. Agent pharmaceutique selon l'une des revendications précédentes 2 à 8,
**caractérisé en ce que**
le suppositoire contient 0,02 g de cis-oxoplatine, 0,02 g de dioxyde de silicium et 1,85 g de graisse solide, ou alternativement, que le suppositoire contient 20 mg de cis-oxoplatine, 1055 mg de lactose, 170 mg d'amidon de maïs, 63,60 mg d'acide adipique, 50 mg d'hydrogénocarbonate de sodium, 5 mg d'acide stéarique, 4,5 mg de stéarate de magnésium, 3 mg de dioxyde de silicium fortement dispersé et 0,5 mg de polysorbate 80, ou alternativement, que le suppositoire contient 20 mg de cis-oxoplatine, 1350 mg de lactose·1H₂O, 170 mg d'amidon de maïs, 65 mg d'acide adipique, 50 mg d'hydrogénocarbonate de sodium, 5 mg d'acide stéarique, 4,5 mg de stéarate de magnésium, 3 mg de dioxyde de silicium fortement dispersé et 0,5 mg de polysorbate 80, ou de façon alternative, que le suppositoire contient 50 mg de cis-oxoplatine, 1450 mg de lactose·1H₂O, 170 mg d'amidon de maïs, 65 mg d'acide adipique, 50 mg d'hydrogénocarbonate de sodium, 5 mg d'acide stéarique, 4,5 mg de stéarate de magnésium, 3 mg de dioxyde de silicium fortement dispersé et 0,5 mg de polysorbate 80.

10. Agent pharmaceutique selon l'une des revendications précédentes 2 à 9,
**caractérisé en ce que**
la préparation d'une solution d'injection ou de perfusion de 5 mg/ml contient 5 mg de cis-oxoplatine, 9 mg d'alcool benzylique, 2 mg de polysorbate 80, 650 mg de solution de sorbitol à 70 % et 500 mg d'eau.

11. Agent pharmaceutique selon l'une des revendications précédentes 2 à 10,
**caractérisé en ce que**
le comprimé contient 50 mg de cis-oxoplatine, 39,5 mg de lactose, 2,5 mg d'amidon de maïs, 2,5 mg de poly(O-carboxyméthyl)amidon-sel de sodium, 2,5 mg de hydrogénophosphate de calcium 2 H₂O, 2,5 mg de poudre de cellulose et 0,5 mg de stéarate de magnésium ou le comprimé contient d'une autre façon 50 mg de cis-oxoplatine, 39 mg de lactose, 2 mg d'amidon de maïs, 2,5 mg de poly(O-carboxyméthyl)amidon-sel de sodium, 2,5 mg de hydrogénophosphate de calcium 2 H₂O, 2,5 mg de poudre de cellulose et 0,5 mg de stéarate de magnésium ou le comprimé contient d'une autre façon 50 mg de cis-oxoplatine, 50 mg de dioxyde de silicium et 50 mg de stéarate de magnésium.

12. Utilisation de l'agent pharmaceutique selon l'une des revendications 2 à 11 pour préparer un médicament destiné à la prophylaxie ou au traitement des tumeurs.
